# EUROPEAN PATENT APPLICATION

(11) **EP 3 078 754 A1**
(43) Date of publication of application: **12.10.2016**
(21) Application number: 16171840.8
(22) Date of filing: 22.09.2011
(51) Int. Cl.: C12Q 1/68

(54) **A METHOD FOR DETECTING, QUANTIFYING AND MAPPING DAMAGE AND/OR REPAIR OF DNA STRANDS**

(30) Priority: 24.09.2010 US 386358 P
(62) Divisional of application: 11802132.8
(71) Applicant: Genomic Vision, 92220 Bagneux (FR)
(72) Inventor: CINQUE, Lucia, 77300 Fontainebleau (FR); BENSIMON, Aaron, 92160 Antony (FR)
(74) Representative: Desaix, Anne

(57) **Abstract**

Methods and products for detecting in vitro the presence of damage on DNA or the presence of a biological response to damage on DNA at the molecular level. Molecular Combing or other nucleic acid stretching methods are employed together with compounds reacting with DNA, probes binding DNA, or nucleic acid monomers, especially labeled nucleic acid monomers.

## Description

### BACKGROUND OF THE INVENTION

### Field of the Invention

Methods and products for detecting *in vitro* the presence of damage on DNA or the presence of a biological response to damage on DNA at the molecular level. Molecular Combing or other nucleic acid stretching methods or similar methods are employed together with compounds reacting with DNA, probes binding DNA, or nucleic acid monomers, especially labeled nucleic acid monomers.

### Description of the Related Art

DNA damage occurs when an alteration or a loss is generated along the DNA molecule. It is important to distinguish DNA damage and mutation. An analogy illustrates the difference: the word "TIME" can be mutated to the word "TIDE" by the substitution of the letter "D" to the letter "M", whereas if the letter "M" is lost or altered, damage occurs, resulting in a no-meaning word: "TI#E". By analogy, substitution of a thymine for an adenine would be a mutation, whereas loss of an adenine or methylation of a guanine would constitute damage. The phenomena are not independent, however, because methylated guanine is known to be mutagenic (Warren, Forsberg et al., 2006) Mutation usually results in transcription that produces proteins with diminished or altered functionality and is likely to be perpetuated in dividing cells. DNA damage interferes with replication and transcription and can dramatically affect the progress of cell cycle if the cell is unable to repair it.

There are more than 200,000 DNA damage events per mammalian cell per day (Saul and Ames, 1986), which are constantly repaired to recover normal cell activity and guarantee cell survival. Damage sources can be endogenous or exogenous with respect to the cell. Endogenous damage is mostly due to oxygen radicals produced during normal cellular respiration, or alkylating and hydrolyzing compounds. Exogenous damage is produced when cells are exposed to a genotoxic agent (an agent that affects the integrity of a cell's genetic material, such as a mutagen or carcinogen). These agents include certain wavelengths of radiation: gamma rays and x-rays, UV-C (∼260 nm) and UV-B rays that penetrate the ozone shield; highly-reactive oxygen radicals produced by external biochemical pathways; chemicals in the environment: hydrocarbons, plant and microbial products, drugs used as therapeutic agents (*e*.*g*., antimicrobials or drugs used for chemotherapy).

The effects of genotoxic or nucleic acid damaging agents at the molecular level can be classified in four main groups: base alterations, mismatches, cross-links and breaks.

All four of the bases in DNA (A, T, C, G) can be covalently modified at various positions, resulting in base loss or covalent alteration. The most frequent lesions result from nucleic acid deamination, depurination, depyrimidation, methylation (7-methylguanine, 1-methyladenine, 6-O-methylguanine) and oxidation (8-hydroxy-2-deoxy guanosine and 8-oxo-7,8-dihydroguanine or 8-oxoG) (Zharkov, 2008). Failure of proof-reading by cellular mechanisms during DNA replication can generate mismatches of the normal bases: a common example is incorporation of the pyrimidine U (normally found only in RNA) instead ofT (Larson, Bednarski et al. 2008) into DNA. Cross-links can be formed between bases on the same DNA strand ([6-4]-PP, pyrimidine dimers) (Pfeifer, 1997) or on opposite strands ("inter-strand cross-links") (McCabe, Olson et al. 2009). Breaks in the backbone can be limited to one of the two strands (single-strand break or "SSB") (Caldecott 2008) or they can cut the molecule on both strands, producing a double-strand break ("DSB") (Shrivastav, De Haro et al. 2008).

Types and background frequency of DNA damage are illustrated in Table 1.

**Table 1: Baseline distribution of DNA damage occurring daily in a eukaryotic cell.**

| Damage Type | # | % |
|---|---|---|
| Single-strand breaks | 120,000 | 50.9 |
| N⁷-MethylGuanine | 84,000 | 35.6 |
| Depurination | 24,000 | 10.2 |
| O⁶-MethylGuanine | 3,120 | 1.3 |
| 8-oxo-7,8-dihydroguanine | 2,880 | 1.2 |
| Depyrimidation | 1,320 | 0.5 |
| Cytosine deamination | 360 | 0.2 |
| Pyrimidine dimers | 200 | 0.1 |
| Double-strand breaks | 9 | 0.01 |
| Interstrand cross-links | 8 | 0.01 |

The outcome of DNA damage is diverse and complex, but generally constitutes a danger for the cell.

Short-term effects arise from blockage of basic operations on DNA, triggering cell-cycle arrest or cell death. Many lesions stall transcription and generate acute transcriptional stress, which constitutes an efficient trigger for p53-dependent apoptosis (Evan and Vousden, 2001). To prevent such mechanisms, cells developed a high-priority repair system called transcription-coupled repair (TCR), which displaces or removes the arrested RNA polymerase and assures quick repair (Tornaletti and Hanawalt 1999).

Long-term effects result from erroneous correction or conversion of lesions causing irreversible mutations and contributing to oncogenesis (Hoeijmakers 2001). It is often the case of lesions that interfere with DNA replication (generally SSB, alkylations, helix distortions). A class of polymerases with flexible base-pairing properties is recruited when damage-induced replicational stress occurs. These recently discovered enzymes take over temporarily from the blocked replicative DNA polymerase and permit trans-lesion synthesis. This process can be beneficial but comes at the expense of a higher error rate. It seems to be responsible for most of damage-induced point mutations and is thus particularly relevant for oncogenesis (Kunkel and Bebenek 2000).

Lesions affecting both strands (DSB and interstrand-crosslinks) are direct cause of recombination. If the genetic information is interrupted, the chromosome integrity cannot be restored with high fidelity and segregation cannot progress properly during mitosis. The consequences are closely associated with carcinogenesis: chromosomal aberrations, including aneuploidy, deletions and chromosomal translocations (Khanna, K. K. and S. P. Jackson 2001).

In order to face all the possible damages that menace DNA integrity and control their impact on the vital genetic information, cells have developed complex repair systems that often interact and support each other.

A spatial extension criterion allows drawing an intuitive picture of how the repair machinery of the cell organizes after a damage checkpoint is activated.

In the case of a lesion located on only one strand, two template-based enzymatic complexes are recruited, depending on spatial extension of the damage: the Base-Excision Repair (BER) targets small chemical alterations of single bases (Lindahl and Wood 1999); the Nucleotide-Excision Repair (NER) recognizes larger, multi-base alterations (de Laat, Jaspers et al. 1999). In a 'cut-and-patch'-type reaction, the injury is taken out and the resulting single-stranded gap is filled in using the intact complementary strand as template. BER is one of the primary methods to correct errors in the base sequence and is therefore particularly relevant for preventing mutagenesis. NER is a flexible system that recognizes different types of helix-distorting lesions, which prevent proper base pairing. NER is composed of two sub-pathways depending on the substrate: global genome NER (GG-NER) surveys the entire genome for helix distortions and transcription-coupled repair (TCR) focuses on distortions that block transcription (Tornaletti and Hanawalt 1999). NER is also recruited to support the MisMatch Repair (MMR) system, which specifically coordinates the replication machinery when an error is introduced during normal DNA replication (Plotz, Zeuzem et al. 2006).

In the case of damage affecting both strands, no template strand is available to perform the repair. As a direct consequence, these lesions are difficult to correct and constitute a real danger for the cell. In such cases, recombinational mechanisms (Homologous Recombination (HR) and End-Junction (EJ)), also involved in other cellular pathways, need to be activated (Shrivastav, De Haro et al. 2008)

Detection of genome injury has to take place rapidly in order to activate specific checkpoints (the so-called SOS response) that arrest cell-cycle and allow repairing the damage before it is converted into permanent mutations (Zhou, Elledge 2000). When damage is too significant (in terms of amount and/or seriousness), the whole cell is sacrificed by initiating apoptosis, in the aim of preventing transmission of mutated genetic material (Evan and Vousden, 2001). Cell fate depends on this delicate balance between damage significance and damage conversion into elements that become permanent, whether they represent mutation or not. This balance is the common paradigm underlying the mechanism of aging and carcinogenesis (Finkel, Serrano et al. 2007). Playing with this balance is what confers therapeutic potential to genotoxic agents, especially ionizing radiations (Asaithamby and Chen 2009). More directly, the biological significance of DNA damage is evidenced by the predisposition to malignancy induced by inherited defects in repair pathways. As an example, deficiency in NER pathways causes severe diseases (Xeroderma Pigmentosum, Cockayne's Syndrome) and predisposition to skin cancer; lack of proper response or repair of DSB is involved in Ataxia Telangiectasia syndrome and directly correlated to lymphoma, breast and ovarian cancer (Hoeijmakers 2001).

Molecular combing is a technique enabling uniform stretching of macromolecules and particularly nucleic acids on a substrate by the action of a moving interface. Molecular combing technology has been disclosed in various patents and scientific publications, for example in US 6,303,296, WO9818959, WO0073503, US2006257910, US2004033510, US6130044, US6225055, US6054327, WO2008028931 and Michalet, Ekong et al. 1997, Herrick, Michalet et al. 2000, Herrick, Stanislawski et al. 2000, Gad, Aurias et al. 2001, Gad, Caux-Moncoutier et al. 2002, Gad, Klinger et al. 2002, Derrick, Jun et al. 2002, Pasero, Bensimon et al. 2002, Gad, Bieche et al. 2003, Lebofsky and Bensimon 2003, Herrick, Conti et al. 2005, Lebofsky and Bensimon 2005, Lebofsky, Heilig et al. 2006, Patel, Arcangioli et al. 2006, Rao, Conti et al. 2007 and Schurra and Bensimon 2009 (See references). Molecular Combing and related substrates have also been used to immobilize nucleic acids on a surface in partially stretched or non-stretched form (Allemand, Bensimon et al. 1997).

Stretching nucleic acid, in particular viral or genomic DNA provides immobilized nucleic acids in linear and parallel strands, and is preferably performed with a controlled stretching factor, on an appropriate surface (*e*.*g*., surface-treated glass slides). It is possible to stretch nucleic acids containing modified monomers (*e*.*g*., biotin-modified nucleotides). Thus, a nucleic acid strand synthesized by a living cell or *in vitro* in the presence of modified nucleotides may be linearized and detected for example by converting modified-nucleotides into fluorescent ones (Herrick, Jun et al. 2002). Moreover, after stretching it is possible to hybridize sequence-specific probes detectable similarly by fluorescence microscopy (Herrick, Michalet et al. 2000). Thus, a particular sequence may be directly visualized, on a single molecule level. The length of the fluorescent signals and/or their number, and their spacing on the slide provides a direct reading of the size and relative spacing of the targeted sequences.

Rudimentary DNA elongation involving non-homogeneous elongation of the DNA has been used to estimate DNA length and assess numbers of lesions in DNA by measuring fluorescent intensity without the use of probes to localize damages in genomic sequences or mapping. However, there has been a need for a highly sensitive and specific way to identify, localize or map damage or repair to DNA that does not primarily rely on measurement of fluorescent intensity and which simultaneously permits visualization of different types of damage.

Considering the implications to health of damage and repair of nucleic acids including assessment of cancer and disease treatment by various agents, or for the development or identification of new agents, it has become critical to develop sensitive and effective methods to characterize and quantify the damaged or repaired state of a nucleic acid, particularly cellular DNA.

### BRIEF SUMMARY OF THE INVENTION

The present invention provides a method for detecting *in vitro* the presence of damage on DNA or the presence of the biological response to damage on DNA at the molecular level. Such a method comprises the use of Molecular Combing or other nucleic acid stretching methods or similar methods (wherein nucleic acid stretching comprises molecule elongation to its contour length or more) together with compounds reacting with DNA, probes binding DNA, or nucleic acid monomers, especially labeled nucleic acid monomers. These methods can provide at the same time sensitivity to low levels of damage and high accuracy in the quantification of both damage and repair capability.

Methods for quantifying *in vitro* the presence of damage on DNA or the presence of the biological response to damage on DNA are also provided. Said quantification can be performed in a direct or indirect manner: in the first case, target features are directly visualized and counted; in the second case, the amount of target features is deduced comparing a sample profile to a reference profile.

These methods may also be used to determine genomic localization of damage to a nucleic acid or to assess responses to damage on DNA by means of DNA hybridization.

Methods for following *in vitro* the presence of damage or response to damage on DNA after treatment with a genotoxic or antimicrobial agent are provided as are companion tests, for example for the evaluation of the efficiency of an antimicrobial or anticancer drug. The methods disclosed are applied as companion tests when the effect of a compound of interest consists in a direct action on the DNA or on a specific repair pathway. They also serve as companion tests when the compound of interest targets a related factor or signaling pathway, who's altered functioning can influence the global response of the cell to genotoxic exposure or to other mechanisms involved in the generation or the persistence of damage within the DNA.

Kits are disclosed which are useful for practicing these methods comprising the elements required to carry out a method of the invention, in particular the elements necessary to detect the targeted lesion or repair on stretched molecules. Said detectable elements can act on the targeted lesion or repair by substituting it, binding to it or converting it into a molecular extremity.

Substitution of target arises when the target feature of the nucleic acid is partially or completely replaced by a detectable element. Examples of detectable elements substituting the target damaged or repaired nucleic acids comprise: chemically modified or labeled nucleotides and nucleosides, carrying a biotin molecule, a digoxigenin molecule, an avidin molecule, an electrical charged transferring molecule, a semiconductor nanocrystal, a semiconductor nanoparticle, a colloid gold nanocrystal, a ligand, a nanobead, a microbead, a magnetic bead, a paramagnetic particle, a quantum dot, a chromogenic substrate, an hapten, an antibody, a fragment of antibody, a lipid, a metal complex, a Rh complex, a Ru complex or any combination thereof. Substitution of a target feature by a detectable element takes place in the presence of one or more enzymes able to excise the lesion from the nucleic acid and replace it by freshly synthesized nucleic acid containing one or more detectable elements. These enzymes are present inside living cells, within cell extracts or can be synthesized in vitro. Excision can also be performed using alkaline solutions or other chemical treatments, followed by enzymatic incorporation of a detectable element into the nucleic acid.

Binding takes place when the detectable element positions and attaches to the nucleic acid in correspondence of the target feature, spontaneously or through the action of an enzyme. Binding can follow covalent modification of the target feature or non-covalent attachments such as antibody-ligand interactions or complexation. The target feature may be chemically modified through the binding but it is not excised from the nucleic acid. Examples of detectable elements binding to the target damaged or repaired nucleic acids comprise: a chemically modified or labeled nucleotide or nucleoside, an antibody, a fragment of antibody, a lipid, a metal complex, a Rh complex, a Ru complex, a molecule capable of reacting chemically with the target feature or any combination thereof. The mentioned substances are detected directly or carry a molecule enabling detection, such as biotin molecule, a digoxigenin molecule, an avidin molecule, an electrical charged transferring molecule, a semiconductor nanocrystal, a semiconductor nanoparticle, a colloid gold nanocrystal, a ligand, a nanobead, a microbead, a magnetic bead, a paramagnetic particle, a quantum dot, a chromogenic substrate, an hapten, an antibody, a fragment of antibody, a lipid, a metal complex, a Rh complex, a Ru complex or any combination thereof.

Conversion into a molecular extremity takes place through cleavage of the nucleic acid molecule in correspondence of the target features. The resulting fragments of the initial nucleic acid molecule have novel molecular extremities, which constitute the detectable elements. Conversion of the target into a molecular extremity can be induced by chemical treatment, heat treatment, enzymatic treatment or any combination thereof. Any type of molecular extremity constitutes a detectable element: duplex nucleic acid extremity, non-duplex nucleic acid extremity, "sticky-ends", restriction enzyme-like extremity, and blunt nucleic acid extremity.

Methods are disclosed for the detection or the diagnosis of damage (alteration or loss) in the structure or sequence of DNA or another nucleic acid. Said damage triggers signaling pathways that lead to an alteration in the normal cell cycle. The present invention concerns the detection of said damage in both cells capable of reestablishing normal cell cycle progress after repair and in cells that lack normal repair activity (*e*.*g*., cancer cells, cells lacking specific repair pathways). The present invention also concerns the detection of the sites on the nucleic acid that have been repaired after damage. The method of the invention enables to follow the repair activity in both normal and abnormal cells, evaluate repair capacity of selected repair systems, measure repair efficiency, kinetics, and influence of environmental factors.

In another aspect, methods are disclosed which enable one to evaluate the effects of a genotoxic agent or a cytotoxic compound on a biological sample, in terms of quantification of damage induced and repaired, and localization of such events on the DNA. The present method is also useful to predict cell death or loss of normal activity due to insufficient or altered repair of damage induced by a genotoxic agent.

Other aspects of the invention include a method for detecting the presence or absence of a repaired, damaged, altered or mutated sequence on a nucleic acid comprising:(a) extracting a one or more nucleic acids from a sample, and optionally rinsing or washing the extracted sample, (b) stretching the least one nucleic acid in said extracted sample, (c) adding a detectable substance to the stretched nucleic acid, which substance positions itself on one or more damaged or repaired portions of the stretched nucleic acid by substituting, binding to it, or converting it into a molecular extremity, (d) detecting the detectable substance on the stretched nucleic acid, and (e) detecting the presence of damaged or repaired nucleic acid when said substance is detected and detecting the absence of a damaged or repaired nucleic acid sequence when said detectable substance is not detected; or (a) extracting a one or more nucleic acids from a sample, and optionally rinsing or washing the extracted sample, optionally rinsing or washing the extracted nucleic acid sample, (b) adding a detectable substance to said nucleic acid for a time and under conditions sufficient for interaction, which substance positions itself on one or more damaged or repaired portions of the stretched nucleic acid by substituting, binding to it, or converting it into a molecular extremity, optionally rinsing or washing the nucleic acid sample after contacting it with the detectable substance, (c) stretching the at least one nucleic acid in said interacted nucleic acid sample,(d) detecting the detectable substance on the stretched nucleic acid, and (e) detecting or diagnosing the presence of damaged or repaired nucleic acid when said substance is detected and detecting or diagnosing the absence of a damaged or repaired nucleic acid sequence when said detectable substance is not detected; or (a) treating a sample containing cells prior to extracting nucleic acids from said sample by adding a detectable substance for a time and under conditions sufficient for interaction with nucleic acids, which substance positions itself on one or more damaged or repaired portions of the nucleic acid by substituting, binding to it, or converting it into a molecular extremity, (b) extracting a one or more nucleic acids from said sample, and optionally rinsing or washing the extracted nucleic acid sample, (c) stretching the at least one nucleic acid in said interacted nucleic acid sample, (d) detecting the detectable substance on the stretched nucleic acid, and (e) detecting or diagnosing the presence of damaged or repaired nucleic acid when said substance is detected and detecting or diagnosing the absence of a damaged or repaired nucleic acid sequence when said detectable substance is not detected.

In the methods described above the substance may position itself on one or more damaged portions of the stretched nucleic acid by substituting, binding to it, or converting it into a molecular extremity; it may position itself on one or more repaired portions of the stretched nucleic acid by substituting, binding to it, or converting it into a molecular extremity; may position itself on one or more damaged or repaired portions of the stretched nucleic acid by substituting; may position itself on one or more damaged or repaired portions of the stretched nucleic acid by binding to it; or position itself on one or more damaged or repaired portions of the stretched nucleic acid by converting it into a molecular extremity.

These methods may further be directed to diagnosing a disease, disorder or condition by detecting a damaged or repaired portion on the nucleic acid; or be directed to diagnosing recovery from a disease, disorder or condition by detecting a damaged or repaired portion on the nucleic acid. These methods may also be used to detect modifications in the genome of test cells cultivated *in vitro* when exposed to an agent or condition that modifies or alters their genomic components.

The samples used in these methods are not particularly limited so long as they contain nucleic acid and include tissue samples, or blood, cerebrospinal fluid, synovial fluid, or lymph samples. The samples may be obtained from subjects in need of diagnosis of a particular disease, disorder or condition including subjects who have genetic diseases or disorders, cancer, infectious disease, autoimmune diseases or inflammatory disorders or who have undergone treatment for these diseases or disorders. The samples may be collected at a particular time or collected longitudinally over a period of time to detect differences between cells that occur due to aging, repeated exposure to particular agents, or other time-dependent changes.

In another embodiment this method will involve (a) hybridizing one or more sequence specific probes corresponding to one or more specific known positions or regions on the nucleic acid, and, optionally, (b) measuring the distance or the spatial distribution between the hybridized probes and detectable elements corresponding to one or more damaged or repaired nucleic acid sequences.

In another embodiment, the invention is directed to process for determining the effect of a test agent or a cytotoxic compound on a nucleic acid sequence in a cell encompassing contacting the cell with said test agent for a time and under conditions sufficient for it to repair, damage, alter, or mutate nucleic acid in the cell, and detecting a repaired, damaged, altered or mutated nucleic acid of said cell by the method of claim 1; wherein repaired, damaged, altered or mutated nucleic acid may be assessed by comparison to nucleic acid in an otherwise identical cell not exposed to said test agent. This method may further comprise selection of a test agent that repairs, damages, alters, or mutates a nucleic acid in the cell or a second agent that inhibits, enhances or modifies the actions of such a test agent. A test agent may be a genotoxic compound or a physical agent such as ionizing radiation such as UV, X-rays or gamma rays. The methods described herein are generally practiced with eukaryotic cells and DNA extracted from such cells, though it is possible to use other kinds of cells or nucleic acids. In the application of the method, comparison is usually performed between a control cellular sample or a control molecular sample, containing the control nucleic acids, and a test cellular sample or a test molecular sample, containing the altered nucleic acid. In order to evaluate the effect of a test agent, control nucleic acids are usually obtained from the same type and the same amount of cells that are contained in the test sample, belonging to the same individual, extracted from a the same type of tissue but not exposed to the test agent. In order to evaluate the intrinsic damage susceptibility or repair capacity of a test sample, the control nucleic acids are usually obtained from the same type and the same amount of cells that are contained in the test sample, but isolated from apparently healthy tissue of the same type and possibly belonging to the same individual or to individuals of similar age and race. Pertinent criteria enabling the distinction of controls from altered nucleic acids include, but are not limited to: amount of detected target features, at a particular time or longitudinally over a period of time; local molecular distribution of detected target features, at a particular time or longitudinally over a period of time; correlation between local molecular distribution and persistence/disappearance over time of detected target features; genomic position of detected target features; correlation between genomic position and persistence/disappearance over time of detected target features; correlation between cellular processes and persistence/disappearance over time of detected target features; correlation between local molecular structure of the nucleic acid and persistence/disappearance over time of detected target features; correlation between nuclear organization of the nucleic acid and persistence/disappearance over time of detected target features, where a target feature may correspond to a lesion, a damage event, a repaired lesion or a biological response to a damage event found within the studied nucleic acid samples.

Another embodiment is a process of prevention, prophylaxis, treatment or therapy of an organism or host comprising the administration of a test agent selected by the methods described above. As mentioned above, such an organism or host will usually be a eukaryotic organism.

Kits comprising one or more ingredients useful for practicing the method described herein are also contemplated and will contain at least one detectable element which positions itself on one or more damaged or repaired portions of a stretched nucleic acid by substituting, binding to it, or converting it into a molecular extremity; one or more reagents suitable for visualizing the at least one detectable element; and one or more probes that bind to specific locations on a nucleic acid; and, optionally, one or more reagents used for stretching a nucleic acid.

### BRIEF DESCRIPTION OF THE DRAWINGS

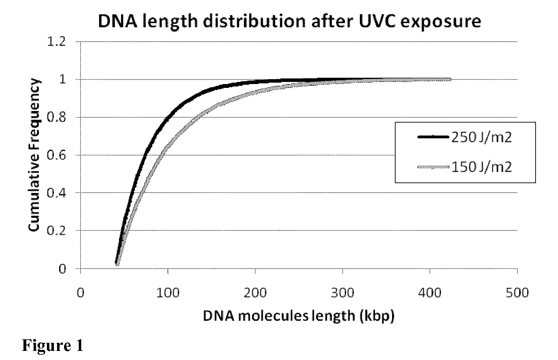

**Figure 1** : Distribution of combed DNA molecules length after UV-C exposure. The length of DNA molecules after Molecular Combing is influenced by the number of "fragile regions" along the DNA, as these increase the probability of DNA fragmentation during manipulation. The damage induced by UV exposure creates such "fragile sites" in a dose-dependent manner. The curves show the effects of this phenomenon: when UV dose increases (from 150 (gray) to 250 J/m² (black)) the length of combed DNA molecules is progressively reduced.

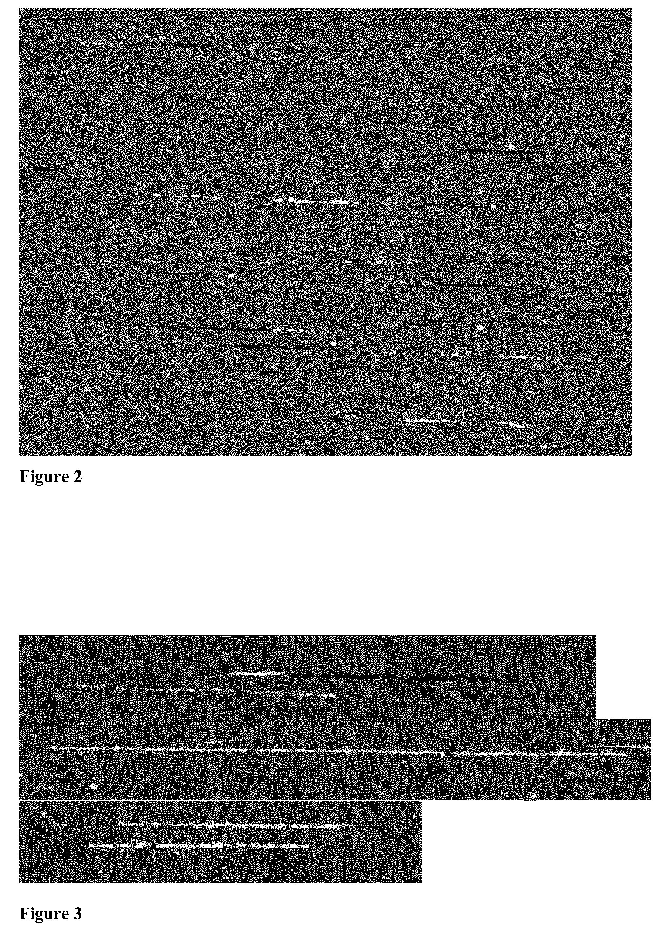

**Figure 2**: Example of replication signals observed on combed DNA extracted from human normal fibroblasts after 30 min incubation with BrdU (5-bromo-2'-deoxyuridine, in white) and 30 min incubation with EdU (5-ethynyl-2'-deoxyuridine, in black). BrdU is detected using a two layers immunofluorescence technique, while EdU is detected by chemical reaction with the fluorophores (appearing in black). White signals from antibodies appear discontinuous when compared to black signals produced by the chemical detection. Moreover, the non-specific adsorption of fluorescent antibodies produces a spotty background, which is not observed for the black channel.
**Figure 3**: Example of EdU signals (appearing in black) observed on combed DNA extracted from human normal fibroblasts after UV-C exposure and 1-hour incubation with EdU. Linear black signal corresponds to DNA replication and spotty black signal to UDS (Unscheduled DNA Synthesis). DNA is stained with YOYO-1 dye (appearing in white).

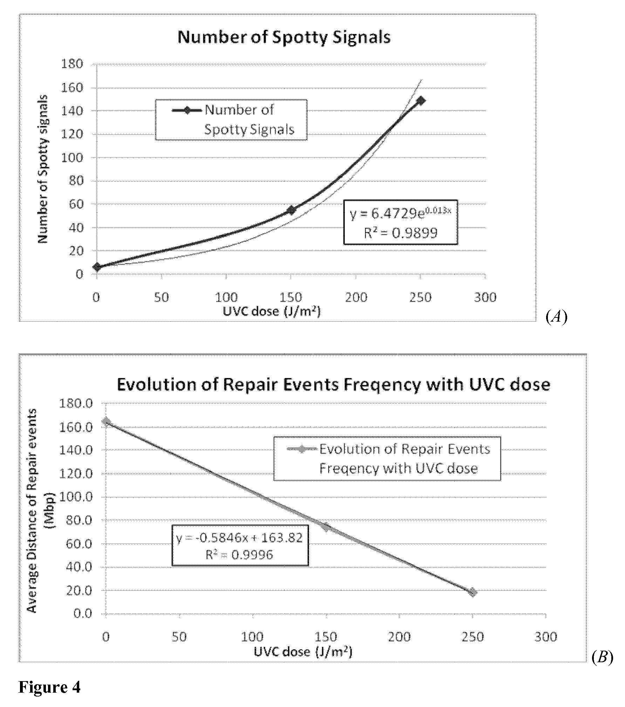

**Figure 4** **:** Evolution of the number of spotty signal and of their average distance on the genome with increasing UV-C exposure. The number of UDS sites increses exponentially with the dose of UV-C (*A*)*,* as confirmed by the linear decrease of their average distance on the combed DNA molecules (*B*). Nucleotide-Excision Repair Capacity (NERCA) appears constant up to the dose of UV-C tested. A deviation from the linear profile is interpreted as a variation in the NERCA of the studied cellular sample.

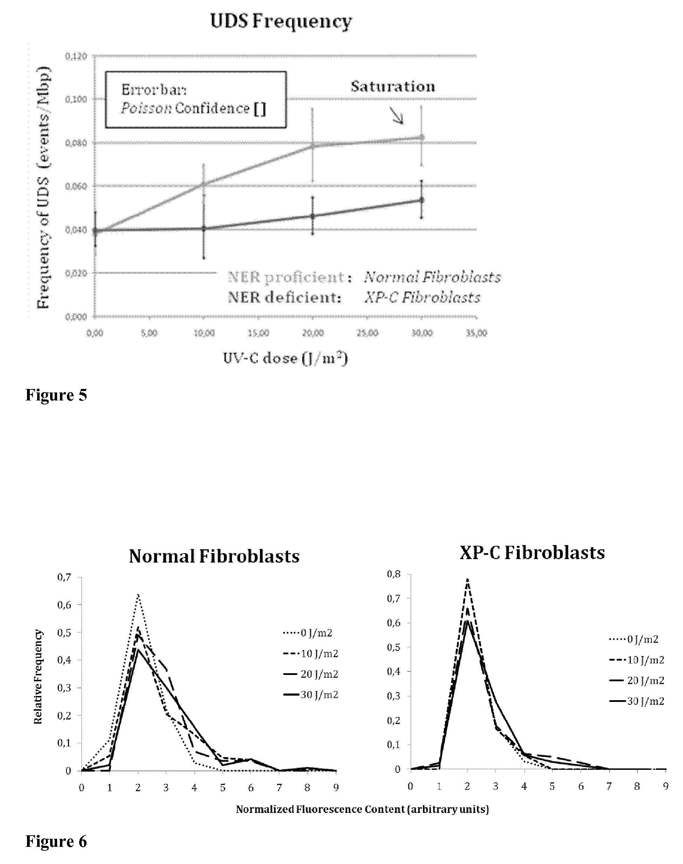

**Figure 5** **:** Evolution of the frequency of UDS signals detected on combed DNA extracted from normal human fibroblasts HS 707(B) and Nucleotide-Excision Repair-deficient fibroblasts XP17BE exposed to four doses of UV-C light. The occurrence of a UDS event was modeled as a Poisson variable. Error bars correspond to Poisson confidence limits. Saturation in the frequency of repair events is observed for the repair-proficient sample after a critical 20 J/m² dose of UV-C.
**Figure 6** **:** Evolution of UDS patch size with UV-C dose. Graphical representation of the distributions of the fluorescence content of UDS signals after internal normalization for normal fibroblasts (left) and for XP-C cells (right). The histograms are plotted in the form of continuous curves to facilitate visualization.

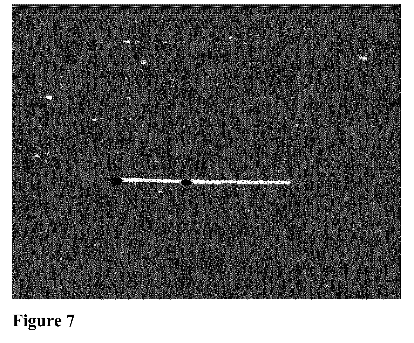

**Figure 7** **:** Detection of molecular extremities (associated to DSB) and SSB on combed lambda phage DNA. 3'-OH ends were pre-labeled for 3h using TdT enzyme and BrdUTP. Tail size is estimated at 150-200 BrdUTP monomers. Black signal is produced by fluorescently labeled anti-BrdU antibodies. DNA is stained with YOYO-1 (appearing in white).

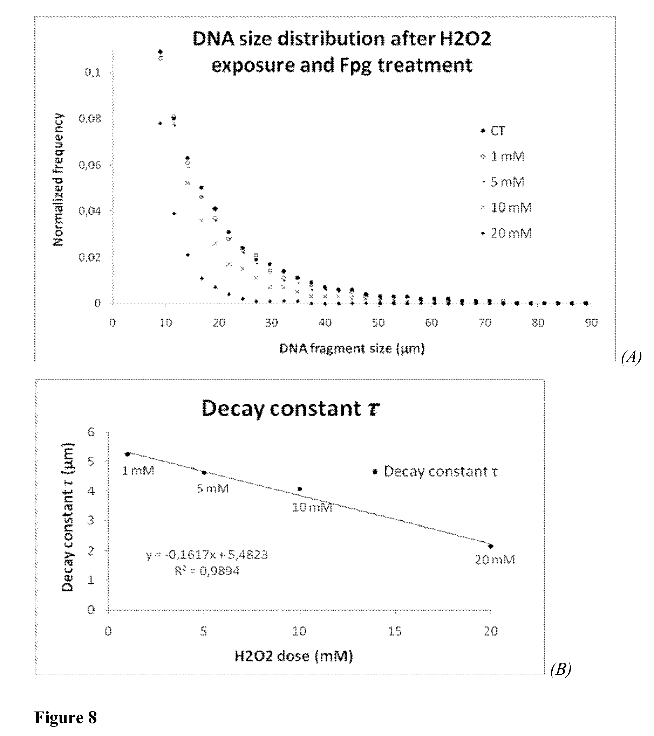

**Figure 8****:** Relative fragmentation Profile Deviation revealed by Formamidopyrimidine-DNA glycosylase (Fpg) enzyme treatment on 5 samples of human DNA extracted from cells exposed to incremental doses of H₂O₂. The left figure shows the distribution of the sizes (x-axis) of DNA fragments in each sample. The distributions were fitted using decaying exponentials y=Ae^{-x/τ} and the evolution of the decay constants τ is illustrated in the right graph.

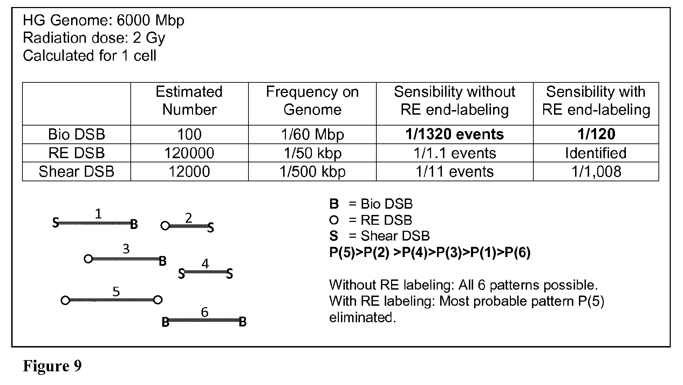

**Figure 9****:** Theoretical approach for the estimation of the amount of DSB generated by exposure to ionizing radiation. To facilitate the identification of radiation induced molecular extremities (Bio DSB); the random fragmentation of DNA induced by manipulation (Shear DSB) is reduced to minimum by the introduction of a Restriction Endonucleases (RE) digestion step. RE digestion allows reducing DNA size under the critical size of shearing and RE extremities (RE DSB) can be labeled in order to be identified unequivocally after DNA stretching. The few remaining unidentified extremities contain the original DSB induced by radiation, which can be quantified comparing to the reference profile.

### DETAILED DESCRIPTION OF THE INVENTION

High sensitivity assays are crucial for general biomonitoring studies. The basal level of DNA damage is influenced by a variety of lifestyle and environmental exposures, including exercise, air pollution, sunlight, and diet. Normal living conditions are responsible for constant but low-dose damages on DNA which directly impact on cellular processes. In this context, the method of the invention is a valuable solution for the detection of genotoxic exposure in humans and provides an effective tool for the characterization of compounds and hazards in public risk assessment. High sensitivity is also required in the field of dermatology and cosmetics: unlike other organs, skin is in direct contact with the environment and therefore undergoes aging as a consequence of environmental damage. The primary environmental factor that causes human skin aging is UV irradiation from the sun. Skin photoaging and photosensitivity are correlated with skin cancer development and have become a social issue for several countries. Better characterization of skin types and response to sun exposure is needed to develop more efficient UV-protective screens. In the Example 2, the method of the invention is successfully used to visualize the repair of single or grouped damage events occurred in normal skin cells exposed to UV radiation, at a resolution never achieved before.

Chemotherapy and radiotherapy aim at generating DNA damage with high significance, in order to selectively induce cancer cells to death, taking advantage of their defective repair systems. The method of the invention, enabling rapid and precise quantification of damaged/repaired sites ratio, represents a precious tool for therapy response prediction and patient follow-up. Precise evaluation of damage would represent just a start in the complex field of personalized therapy: the relationship between molecular response to damage and cellular response is complex and depends on a number of factors beside damage significance: cell type (type of normal tissue, cancer), time of cell cycle, cellular environment (Gerweck, Vijayappa et al. 2006). A second degree of complexity is introduced by individual variability, which is really wide in this field and makes correlation of cellular response to clinical response hard job (Stausbol-Gron and Overgaard 1999).

Similarly to cancer therapy, DNA damage is the cytotoxic target of many antimicrobial drugs. The most important example is the large family of the fluoroquinolones, which are the only direct inhibitors of microbial DNA synthesis. Fluoroquinolones act by binding to the enzyme-DNA complex and stabilize DNA strand breaks created by DNA gyrase and topoisomerase IV (Hooper, 2001). Because resistance to antimicrobial drugs is widespread, an understanding of their mechanisms of action and a precise quality control are vital. In this context too, the method of the invention proves valuable for the precise evaluation of antimicrobials efficiency, specificity, and for the study of the factors involved in the development of drug resistance.

The method of the invention is particularly interesting for its potentiality to detect and analyze different types of damage or damage response at the same time. At present, no single test is able to describe DNA damage completely, due to the variety of DNA lesions and the fact that they are targeted by different mechanisms and have different mutagenic potential and repair kinetics. Two main approaches exist to assay DNA damage: the first group includes techniques that identify specific types of DNA lesions; the second group comprises biochemical assays focused on the measurement of the effects of DNA damage.

Examples of the first group comprise the usage of DNA sizing techniques combined to selective enzymatic treatment targeting specific DNA lesions and converting them into DSB or SSB (Collins 2004). The resulting DNA fragmentation profile can be obtained by traditional techniques like Southern Blot (Bohr, Smith et al. 1985), Pulse-Field-Gel-Electrophoresis (Cedervall, Wong et al. 1995) or more high-throughput means like fluorescent screening on a microchip (Filippova, Monteleone et al. 2003). These techniques lack sensitivity for the detection of low frequency events (like DSB) due to their bulk nature (average signal of a pool of molecules coming from more than one cell). Moreover, fragment sizes are extrapolated from fluorescence intensity measurement, which reduces dramatically sizing accuracy and precision. Finally, DNA fragments are manipulated in the coiled form, which prevents localization studies.

Single-cell gel electrophoresis (SCGE, also called Comet assay) is a versatile tool that allows evaluating low levels of damage on a single cell base. Cells are spread on a surface and embedded in agarose gel. Cell membranes are permeabilized in the presence of a compound that stains DNA and an electric field is applied. The basic concept is that damaged DNA (in particular locally broken strands) can relax and migrate when the electric field is applied while undamaged DNA preserves its organization on compacting proteins and does not leave the nucleus. The cells observed by fluorescence microscopy look like "comets", whose tail size corresponds to the amount of DNA that leaves the cavity and is a measure of the amount of DNA damage in the cell (Ostling and Johanson 1984). When coupled to chemical (Singh, McCoy et al. 1988) or enzymatic treatments (Collins, Dobson et al. 1997), SCGE can provide high sensitivity and specificity to certain types of damage. The drawback is its qualitative character: the extent of DNA damage is estimated by visual or software-aided comparison of the fluorescence intensity in the comet head (undamaged DNA) and in the tail (damaged DNA) (Collins 2004).

Immunochemistry assays have recently allowed direct visualization and quantification of low levels of DSB inside fixed cells. The most widely employed is the immunodetection of the γ-phosphorylated form of histone H2AX, which is known to form very quickly when a DSB is generated (Rogakou, Pilch et al. 1998). These techniques are the most sensitive and direct, but they are complicated to perform from an experimental point of view and unsuitable for clinical use.

Abasic sites can be directly labeled with haptens like biotin via a chemical reaction with Aldehyde Reactive Probe (ARP) reagents (Nakamura, Walker et al. 1998; Kurisu, Miya et al. 2001). Base lesions (e.g., 8-OH-Guanine) can be detected by high-performance liquid chromatography (HPLC) or via GC-mass spectrometry (Dizdaroglu 1984).

The second group of methods generally targets mechanisms that are blocked by the presence of damage on DNA. By consequence, they give often insights on the biological relevance of the investigated damage and its consequences in terms of mutagenesis.

Long-range PCR assays extrapolate damage amount and impact by measuring the reduction of the amplification in a representative sequence. When a polymerase encounters a lesion in the monitored sequence, amplification stops. The result is damage-proportional reduction of amplification efficiency (Lisby, Gniadecki et al. 2005). Their sensitivity depends on the amount of damage and on the limitations of the PCR technique.

Mutagenesis assays often rely on cell transfection with a plasmid containing a reporter gene. Damages converted into mutations will cause gene silencing with a decrease in the amount of gene product as a result (White and Sedgwick 1985). These methods allow evaluating the biological relevance of the damages induced to the cell and can provide models to study recombinational repair; however they can only be performed *in vitro* limiting the possibility of using them on human tissue.

Detection of repair of specific damages can be performed in a direct way when it implies Unscheduled DNA Synthesis (UDS), *i.e.,* for BER and NER systems. Unscheduled DNA Synthesis (UDS) refers to the synthesis of DNA occurring as a specific, local response induced by the presence of an alteration in the structure of the DNA molecule. The patches produced during UDS are defined as unscheduled in order to distinguish them from normal replicated DNA, which is considered as cellular scheduled activity. In the case of BER, test is usually performed *in vitro* using reconstituted proteins and radiolabeled nucleotides. Radioactivity counts allow estimating the number of base lesions that have been repaired (Srivastava, Berg et al. 1998). *In vivo* assays require systems using plasmids, usually carrying one chemically defined lesion (Sattler, Frit et al. 2003). In the case of NER patches, the assays are performed in living cells, providing them with labeled nucleosides of different types as described in more detail below in Example 2.

In many cases, repair capabilities are indirectly deduced studying the evolution of damaged sites over different repair times.

The method of the invention is more comprehensive and flexible than the mentioned assays since it enables both direct and indirect detection of damage and repair at once. Specific damage and repair can be studied and quantified by combining three different strategies on stretched DNA molecules. With the first approach, detection of lesion or repair is performed through partial or complete substitution of the target (*i.e.,* selected alteration of the DNA molecule) by a detectable element; the second approach relies on a detectable element that specifically binds to the target and the third approach consists in the transformation of the target into a molecular extremity. Detailed experimental procedures for the application of these three strategies are provided respectively in Examples 2, 3 and 4.

The method of the invention enables to assess the detection of multiple lesions or repaired lesions on DNA molecules reliably, in a time- and cost-effective fashion, and with small amounts of various starting material: commercial DNA, viral particles, parasites, prokaryotic cells, cultured eukaryotic cells, blood and tissue biopsy from a eukaryotic organism or host (*i.e*., plants, fungi or animals including humans). Molecular Combing is a powerful technique enabling the direct visualization of individual molecules and has been successfully used for the study of replication kinetics by direct visualization of freshly synthesized DNA (Herrick, Conti et al. 2005) and for genome mapping studies (Conti, Bensimon 2002). The use of nanochannel-mediated DNA elongation as a tool for investigating DNA damage and repair has been suggested to provide an alternative to fragment sizing techniques like PFGE or fluorescence intensity (WO/2008/121828). Unlike Molecular Combing, all these sizing methods based on PFGE, single-molecules flowing into micro- (Filippova, Monteleone et al. 2003) or nanochannels (Tegenfeldt, Prinz et al. 2004) do not allow post-processing of the DNA molecules and hybridization studies are very difficult to perform. Moreover, these methods do not provide or provide only partial elongation of DNA molecules. As a consequence, DNA fragments length has to be estimated from fluorescence intensity measurements, which reduces resolution and measurement precision comparing to DNA stretching techniques. Nothing anticipates the use of Molecular Combing or other DNA stretching techniques as tools for high resolution, direct visualization and genomic localization of DNA sites where damage and biological repair occurred. Cao et al. were looking to construct specific fragmentation profiles by converting damaged sites into DSB and measuring the length of the resulting partially elongated DNA fragments (WO/2008/121828, US 7,670,770). None of these publications mentioned the possibility either to directly detect and count damaged sites or repaired sites on stretched nucleic acids or to indirectly estimate the amount of damage by determining the size distribution of the stretched fragments. Furthermore, nothing suggested the application of DNA hybridization on elongated or stretched DNA to investigate the distribution of damage and repair with respect to genome sequence or chromatin organization.

The inventors have shown that Molecular Combing, allowing high resolution analysis of stretched nucleic acid, can be successfully applied to the direct and indirect detection, quantification and genomic localization of the presence of damaged sites (alterations or losses in the sequence or structure of a nucleic acid) and repaired sites (damaged sites reconverted to the original form or to a normal form by the cellular systems) on stretched DNA molecules, which was never suggested before. The method of the invention involving DNA stretching, and particularly Molecular Combing, is the only one method up to now to allow direct visualization, precise quantification and localization of events distributed on DNA at random distances (from 1 base to several millions of bases) with a resolution of at least 500 bp due to the present optical limit of fluorescence imaging.

The principles of the present invention cannot be limited by the limitations of the presently existing method of fluorescence labeling and detection.

Specific aspects of the invention include a method for detecting the presence or absence of a repaired, damaged, altered or mutated sequence on a nucleic acid comprising (a) extracting a one or more nucleic acids from a sample, and optionally rinsing or washing the extracted sample, (b) stretching the least one nucleic acid in said extracted sample, (c) adding a detectable substance to the stretched nucleic acid, which substance positions itself on one or more damaged or repaired portions of the stretched nucleic acid by substituting, binding to it, or converting it into a molecular extremity, (d) detecting the detectable substance on the stretched nucleic acid, and (d) detecting the presence of damaged or repaired nucleic acid when said substance is detected and detecting the absence of a damaged or repaired nucleic acid sequence when said detectable substance is not detected. Alternatively, such a method may comprise (a) extracting a one or more nucleic acids from a sample, and optionally rinsing or washing the extracted sample, optionally rinsing or washing the extracted nucleic acid sample, (b) adding a detectable substance to said nucleic acid for a time and under conditions sufficient for interaction, which substance positions itself on one or more damaged or repaired portions of the stretched nucleic acid by substituting, binding to it, or converting it into a molecular extremity, optionally rinsing or washing the nucleic acid sample after contacting it with the detectable substance, (c) stretching the at least one nucleic acid in said interacted nucleic acid sample,(d) detecting the detectable substance on the stretched nucleic acid, and (e) detecting or diagnosing the presence of damaged or repaired nucleic acid when said substance is detected and detecting or diagnosing the absence of a damaged or repaired nucleic acid sequence when said detectable substance is not detected. Alternatively, such a method may comprise (a) treating a cellular sample prior to extracting nucleic acids from said sample by adding a detectable substance for a time and under conditions sufficient for interaction with nucleic acids, which substance positions itself on one or more damaged or repaired portions of the nucleic acid by substituting, binding to it, or converting it into a molecular extremity, (b) extracting a one or more nucleic acids from said sample, and optionally rinsing or washing the extracted nucleic acid sample, (c) stretching the at least one nucleic acid in said interacted nucleic acid sample, (d) detecting the detectable substance on the stretched nucleic acid, and (e) detecting or diagnosing the presence of damaged or repaired nucleic acid when said substance is detected and detecting or diagnosing the absence of a damaged or repaired nucleic acid sequence when said detectable substance is not detected. Both of the above methods may employ a substance that positions itself on one or more damaged portions of the stretched nucleic acid by substituting, binding to it, or converting it into a molecular extremity. These methods may comprise specific steps such as hybridizing one or more sequence specific probes corresponding to one or more specific known positions or regions on the nucleic acid, and/or measuring the distance or the spatial distribution between the hybridized probes and detectable elements corresponding to one or more damaged or repaired nucleic acid sequences.

These methods may also comprise diagnosing a disease, disorder or condition by detecting a damaged or repaired portion on the nucleic acid; or comprise diagnosing recovery from a disease, disorder or condition by detecting a damaged or repaired portion on the nucleic acid. Damage to nucleic acid as a result of aging may also be assessed or used forensically to determine the age of a subject.

Samples containing nucleic acids are used in the method and may be obtained from an *in vitro* source or *in vivo* such as from a tissue sample, or blood, cerebrospinal fluid, synovial fluid, or lymph of a subject. Subjects may be normal subjects or those having diseases or disorders such as cancer, infectious disease, autoimmune disease, or inflammatory disease, or prior or ongoing treatment for these diseases or disorders. Samples may also be acquired over a period of time, for example, to assess treatment-related or aging-related alterations in cellular nucleic acids. Samples may be expanded, processed or treated prior to extraction of nucleic acids.

A process for determining the effect of a test agent on a nucleic acid sequence in a cell comprising contacting the cell with said test agent for a time and under conditions sufficient for it to repair, damage, alter, or mutate nucleic acid in the cell, and detecting a repaired, damaged, altered or mutated nucleic acid of said cell by the methods described herein; wherein repaired, damaged, altered or mutated nucleic acid may be assessed by comparison to nucleic acid in an otherwise identical cell not exposed to said test agent. Such methods may be used to identify new agents or identify and select from the pool or existing agents for one that repairs, damages, alters, or mutates a nucleic acid in a particular cell. Representative cells include eukaryotic cells, mammalian cells, including those of domestic animals or livestock, and humans. The methods herein may be practiced with different nucleic acids. Generally DNA samples will be more conveniently used due to their stability. The effects of genotoxic, genoreparative or stabilizing agents, including chemical compounds or various forms of physical agents, such as genotoxic ionizing radiation, may be screened at the molecular level and their final outcome on the cellular processes may be related to their mechanisms of action on the nucleic acids. Agents identified and characterized by the methods disclosed herein may be used to treat a subject.

Kits comprising one or more ingredients useful for practicing the methods disclosed herein may be formulated, optionally with instructions about how to use them to practice these methods and appropriate containers and packaging materials for the components they contain. These kits may contain elements needed to perform the different steps of the methods, such as a combination comprising at least one detectable element which positions itself on one or more damaged or repaired portions of a stretched nucleic acid by substituting, binding to it, or converting it into a molecular extremity; one or more reagents suitable for visualizing the at least one detectable element; and one or more probes that bind to specific locations on a nucleic acid; and, optionally, one or more reagents used for stretching a nucleic acid.

Another aspect of the invention concerns a test for the detection of DNA damage and repair in vitro or ex vivo according to any of the methods described in the present invention.

The following numbered embodiments represent particular embodiments of the methods disclosed herein:
Embodiment 1. A method for detecting the presence or absence of a repaired, damaged, altered or mutated sequence on a nucleic acid comprising:
   (a) extracting a one or more nucleic acids from a sample, and optionally rinsing or washing the extracted sample,
   (b) stretching the at least one nucleic acid in said extracted sample or immobilizing at least one nucleic acid on a substrate in a non-stretched condition,
   (c) adding a detectable substance to the stretched nucleic acid, which substance positions itself on one or more repaired, damaged, altered, or mutated portions of the stretched nucleic acid by substituting, binding to it, or converting it into a molecular extremity,
   (d) detecting the detectable substance on the stretched nucleic acid, and optionally
   (e) detecting the presence of repaired, damaged, altered, or mutated nucleic acid when said substance is detected and detecting the absence of a damaged or repaired nucleic acid sequence when said detectable substance is not detected;
   or
   (a) extracting at least one nucleic acid from a sample, and optionally rinsing or washing the extracted sample, optionally rinsing or washing the extracted nucleic acid sample,
   (b) adding a detectable substance to said nucleic acid for a time and under conditions sufficient for interaction, which substance positions itself on one or more repaired, damaged, altered or mutated portions of the one or more nucleic acids by substituting, binding to it, or converting it into a molecular extremity, optionally rinsing or washing the nucleic acid sample after contacting it with the detectable substance,
   (c) stretching the at least one nucleic acid in said interacted nucleic acid sample or immobilizing the at least one nucleic acid on a substrate in a non-stretched condition,
   (d) detecting the detectable substance on the stretched or immobilized nucleic acid, and
   (e) detecting or diagnosing the presence of repaired, damaged, altered, or mutated nucleic acid when said substance is detected and detecting or diagnosing the absence of a damaged or repaired nucleic acid sequence when said detectable substance is not detected;
   or
   (a) treating a sample containing cells prior to extracting nucleic acids from said sample by adding a detectable substance for a time and under conditions sufficient for interaction with nucleic acids, which substance positions itself on one or more repaired, damaged, altered, or mutated portions of the nucleic acid by substituting, binding to it, or converting it into a molecular extremity,
   (b) extracting a one or more nucleic acids from said sample, and optionally rinsing or washing the extracted nucleic acid sample,
   (c) stretching or immobilizing on a substrate the at least one nucleic acid in said interacted nucleic acid sample,
   (d) detecting the detectable substance on the stretched or immobilized nucleic acid, and
   (e) detecting or diagnosing the presence of repaired, damaged, altered, or mutated nucleic acid when said substance is detected and detecting or diagnosing the absence of a damaged or repaired nucleic acid sequence when said detectable substance is not detected.
Embodiment 2. The method of embodiment 1 which comprises:
   (a) extracting a one or more nucleic acids from a sample, and optionally rinsing or washing the extracted sample,
   (b) stretching the at least one nucleic acid in said extracted sample,
   (c) adding a detectable substance to the stretched nucleic acid, which substance positions itself on one or more repaired, damaged, altered, or mutated portions of the stretched nucleic acid by substituting, binding to it, or converting it into a molecular extremity,
   (d) detecting the detectable substance on the stretched nucleic acid, and
   (e) detecting the presence of repaired, damaged, altered, or mutated nucleic acid when said substance is detected and detecting the absence of a repaired, damaged, altered or mutated nucleic acid sequence when said detectable substance is not detected.
Embodiment 3. The method of embodiment 1 which comprises:
   (a) extracting a one or more nucleic acids from a sample, and optionally rinsing or washing the extracted sample, optionally rinsing or washing the extracted nucleic acid sample,
   (b) adding a detectable substance to said nucleic acid for a time and under conditions sufficient for interaction, which substance positions itself on one or more repaired, damaged, altered or mutated portions of the stretched nucleic acid by substituting, binding to it, or converting it into a molecular extremity, optionally rinsing or washing the nucleic acid sample after contacting it with the detectable substance,
   (c) stretching the at least one nucleic acid in said interacted nucleic acid sample,
   (d) detecting the detectable substance on the stretched nucleic acid, and
   (e) detecting or diagnosing the presence of repaired, damaged, altered, or mutated nucleic acid when said substance is detected and detecting or diagnosing the absence of a repaired, damaged, altered or mutated nucleic acid sequence when said detectable substance is not detected.
Embodiment 4. The method of embodiment 1 which comprises:
   (a) treating a sample containing cells prior to extracting nucleic acids from said sample by adding a detectable substance for a time and under conditions sufficient for interaction with nucleic acids, which substance positions itself on one or more repaired, damaged, altered or mutated portions of the nucleic acid by substituting, binding to it, or converting it into a molecular extremity,
   (b) extracting a one or more nucleic acids from said sample, and optionally rinsing or washing the extracted nucleic acid sample,
   (c) stretching the at least one nucleic acid in said interacted nucleic acid sample,
   (d) detecting the detectable substance on the stretched nucleic acid, and
   (e) detecting or diagnosing the presence of repaired, damaged, altered or mutated nucleic acid when said substance is detected and detecting or diagnosing the absence of a repaired, damaged, altered or mutated nucleic acid sequence when said detectable substance is not detected.
Embodiment 5. The method of any of embodiments 1 to 4, further comprising diagnosing a disease, disorder or condition by detecting at least one repaired, damaged, altered or mutated portion on the nucleic acid; and/or further comprising diagnosing recovery from a disease, disorder or condition by detecting at least one repaired, damaged, altered or mutated portion on the nucleic acid.
Embodiment 6. The method of any of embodiments 1 to 5, wherein said stretching of the at least one nucleic acid is performed using Molecular Combing.
Embodiment 7. The method of any of embodiments 1 to 6, wherein the target is unscheduled DNA synthesis and said stretching of said at least one nucleic acid is substituted by immobilizing single nucleic acid molecules on a substrate in a non-stretched condition.
Embodiment 8. The method of embodiment of any of embodiments 1 to 7, wherein said sample (a) is a tissue sample, or blood, cerebrospinal fluid, synovial fluid, or lymph sample.
Embodiment 9. The method of any of embodiments 1 to 8, wherein said sample (a) is obtained from a subject who has cancer or who has undergone treatment for cancer.
Embodiment 10. The method of any of embodiments 1 to 8, wherein said sample (a) is obtained from a subject having an infectious disease, autoimmune disease, or inflammatory disease or condition.
Embodiment 11. The method of any of embodiments 1 to 10, which comprises:
   (a) hybridizing one or more sequence specific probes corresponding to one or more specific known positions or regions on the nucleic acid, and, optionally,
   (b) measuring the distance or the spatial distribution between the hybridized probes and detectable elements corresponding to one or more repaired, damaged, altered or mutated nucleic acid sequences.
Embodiment 12. A process for determining the effect of a test agent on a nucleic acid sequence in a cell comprising:
   contacting the cell with said test agent for a time and under conditions sufficient for it to repair, damage, alter, or mutate nucleic acid in the cell, and
   detecting a repaired, damaged, altered or mutated nucleic acid of said cell by the method of any of embodiments 1 to 11; wherein repaired, damaged, altered or mutated nucleic acid may be assessed by comparison to nucleic acid in an otherwise identical cell not exposed to said test agent.
Embodiment 13. The process of embodiment 12, wherein said test agent is a genotoxic compound or genotoxic ionizing radiation.
Embodiment 14. A process of treatment or therapy of a eukaryotic organism or host comprising the administration of an agent selected by the method of embodiment 12.
Embodiment 15. A kit comprising one or more ingredients useful for practicing the method of any of embodiments 1 to 11, comprising at least one detectable element which positions itself on one or more damaged or repaired portions of a stretched nucleic acid by substituting, binding to it, or converting it into a molecular extremity; one or more reagents suitable for visualizing the at least one detectable element; and, optionally, one or more probes that bind to specific locations on a nucleic acid; and, optionally, one or more reagents used for stretching a nucleic acid.

The following non-limiting examples describe particular embodiments of the invention.

### Example 1: Quantification of DNA damage and repair capacity in normal human lymphoblasts exposed to UV-C radiation.

UV-C radiation exposure induces non-specific lesions on the DNA molecule, *e*.*g*., SSB, AP sites, oxidized bases etc., together with more specific photoproducts: cyclobutane pyrimidine dimers (CPD) and (6-4)-photoproducts (6-4PP). The following experimental procedure describes in detail the protocol and the materials needed to practice the method of the invention for the simultaneous detection and quantification of a set of damages: in this example, 8-Oxoguanine (indirect), CPDs and 6-4PP (direct immunodetection) and repaired photoproducts (direct UDS labeling). Other types of lesion could be targeted with an analogue experimental procedure.

### Experimental procedures

### Culture of Human Normal Lymphoblasts

Experiments were performed using human normal lymphoblasts, GM17749 cell line at P5. Cells from cryogenized stocks were thawed quickly, and cultured in a T25 culture flask at 37° C, 5% CO₂ to reach a final amount of 2x10⁶ cells. Growth medium used was RPMI 1640 (Roswell Park Memorial Institute medium, Invitrogen) with 15% v/v FBS (Invitrogen) and 2mM L-Glutamine (Gibco, Invitrogen). Cells were incubated 6h with RPMI 1640 containing 0.5% FBS in order to reduce replication before the experiment.

### UV light treatment and UDS labeling

Cell suspension was poured inside four open Petri dishes and two were exposed to UV-C light (UV1 and UV2) produced by a germicidal lamp (Philips, 254 nm, 15 W, 0.8 µW/m²), lid open. Exposure dose was set to 100 J/m², measured with a UV-C radiometer (LT Lutron, ref. Q569239). The two control samples (CT1 and CT2) were simply exposed to air. After exposure, cells were centrifuged at 1000 rpm for 5 minutes. Samples UV1 and CT1 were resuspended in 1X PBS (Phosphate-Buffered Saline, Invitrogen) at a concentration of 5000 cells/µl for immediate preparation of agarose plugs. Samples UV2 and CT2 were resuspended in RPMI 1640 containing 0.5 % v/v FBS and 100 µM 5-ethynyl-2'-deoxyuridine (EdU) (Invitrogen) and cells were incubated for 60 min at 37° C, 5% CO₂ to allow UDS and residual replication labeling.

### Preparation of embedded DNA plugs from cultured cells

After EdU labeling, each cell suspension of samples UV2 and CT2 was mixed with an equal volume of 1X PBS at 4°C, centrifuged at 1000 rpm for 5 minutes at 4°C, rinsed once with 1X PBS 20 at 4°C, centrifuged again at 1000 rpm for 5 minutes at 4°C and resuspended in 1X PBS 20 at a concentration of 5000 cells/µl. For preparation of agarose plugs, cell suspension was then mixed thoroughly at a 1:1 ratio with a 1.2% w/v solution of low-melting point agarose (Nusieve GTG, ref. 50081, Cambrex) prepared in 1 X PBS at 50°C. 90 µL of the cell / agarose mix was poured in a plug-forming well (BioRad, ref. 170-3713) and left to cool down at least 15 min at 4 °C. Lysis of cells in the blocks was performed as previously described (Schurra and Bensimon 2009). Briefly, Agarose plugs were incubated overnight at 50 °C in 250 µL of a 0.5M EDTA (pH 8), 1 % Sarkosyl, 250 µg/mL proteinase K (Eurobio, code : GEXPRK01, France) solution, then washed three times in a Tris 10mM, EDTA 1 mM solution for 30 min at room temperature.

### Cleavage of 8-oxoguanine by hOGG1 treatment

Agarose plugs were transferred into hOGG1 digestion buffer (50mM NaCl, 10mM Tris-HCl, 10mM MgCl_{2,} 1mM DTT) and treated with 1µg/plug of hOGG1 enzyme (New England BioLabs) at 37°C for 3h. Plugs were incubated 1h at 50 °C in 250 µL of a 0.5M EDTA (pH 8), 250 µg/mL proteinase K solution to eliminate residual hOGG1, and then washed three times in a Tris 10mM, EDTA 1 mM solution for 30 min at room temperature.

### Final extraction of DNA and Molecular Combing

Plugs of embedded DNA from human lymphoblasts were treated for combing DNA as previously described (Schurra and Bensimon 2009). Briefly, plugs were melted at 68 °C in a MES 0.5 M (pH 5.5) solution for 20 min, and 1.5 units of beta-agarase (New England Biolabs, ref. M0392S, MA, USA) was added and left to incubate for up to 16h at 42° C. The DNA solution was then poured in a Teflon reservoir and Molecular Combing was performed using the Molecular Combing System (Genomic Vision S.A., Paris, France) and CombiCoverslips (20 mm x 20 mm, Genomic Vision S.A., Paris, France). The combed surfaces were cured for 4 hours at 60 °C.

### Detection of UDS and Replication Signal

Detection of alkyne-labeled nucleotides was performed by Cu(I)-catalyzed Huisgen cycloaddition (Click) reaction as previously described (Salic and Mitchison 2008). Briefly, a reaction mixture of 100 mM Tris Buffer pH 8.5, 0.5 mM CuSO₄ (Sigma), 1 µM Alexa Fluor® 594 azide (Invitrogen) and 50 mM sodium L-ascorbate (Sigma) (added last to the mix from a 0.5 M solution) was freshly prepared and mixed with Block-Aid (Invitrogen, ref. B-10710) in a 1:1 ratio. 20 µl of reaction mixture were poured on top of clean glass slides and covered with a combed surface. Cover-slips were incubated for 30 min at RT protected from light, then rinsed for 1 min in deionised water with agitation at 500 rpm. A second incubation with a newly prepared reaction mixture was performed for other 30 min. Surfaces were then rinsed twice in 1X PBS for 5 minutes and once in deionised water for 1 minute, both with agitation at 500 rpm. Residual water was dried with compressed air.

### Immunochemical detection of CPDs and 6-4PP

Detection was performed using antibody layers. For each layer, 20 µL of the antibody solution was added on the slide and covered with a combed coverslip and the slide was incubated in humid atmosphere at 37 °C for 20 min. The slides were washed 3 times in a 2x SSC, 1 % Tween20 solution for 3 min at room temperature between each layer and after the last layer. Detection was carried out in this example using Mouse Anti-CPDs (CosmoBioCo, Ltd, Clone: TDM-2) and Mouse Anti-6-4PPs (CosmoBioCo, Ltd, Clone: 64M-2) in a 1:25 dilution. As second layer antibody, Alexa Fluor® 350-coupled goat anti mouse (Invitrogen, France) diluted at 1:25 was used.

### Analysis of Fluorescent Signals

For direct visualization of combed DNA, cover-slips were mounted with 20 µL of a Block-Aid (Invitrogen, ref. B-10710)-YOYO-1 iodide (Molecular Probes, code Y3601) mixture (10000:1 v/v) in order to counter-stain all stretched molecules. Imaging was performed with an inverted automated epifluorescence microscope, equipped with a 40X objective (ImageXpress Micro, Molecular Devices, USA). Length of the YOYO-1-stained DNA fibers were measured and converted to kb using an extension factor of 2 kb/µm (Schurra and Bensimon 2009), with an internal software GVlab v0.4.6 (Genomic Vision S.A., Paris, France). Length distribution histograms were constructed from DNA measurements and compared for the indirect evaluation of the amount of 8-Oxoguanine lesions present in the samples just after UV exposure (UV1 against CT1) and after 1h repair (UV2 against CT2). A total amount of 20 Gbp DNA was analyzed. Specific blue signals and red signals, corresponding respectively to anti-photoproducts antibodies and UDS, were identified when positioned on YOYO-1 stained DNA molecules and then quantified as number of events/kbp. Comparison of samples UV1 and UV2 normalized to their controls allowed evaluating the short-term repair capacity of the lymphoblasts sample.

### Example 2: Detection of lesion or repair through partial or complete substitution of target by a detectable element.

### Detection oƒ NER Repair Capacity (NERCA)

The Nucleotide Excision Repair (NER) system is a versatile enzymatic DNA repair pathway involved in the repair of a broad variety of structural DNA lesions with DNA helix distorting properties (van der Wees, Jansen et al. 2007) including UV-induced cyclobutane pyrimidine dimers (CPD) and (6-4)-photoproducts (6-4PP). Two NER sub-pathways have been identified, *i.e.,* global genome repair (GGR) and a specialized pathway termed transcription coupled repair (TCR). GGR removes DNA lesions throughout the genome, whereas TCR targets lesions in the transcription of active genes (Fousteri, Mullenders 2008).

NER is a complex multistep repair process involving more than 30 polypeptides. The XPC-hHR23B heterodimer is the principal damage recognition factor in GGR and is strictly required for the recruitment of all following NER proteins to the damaged DNA. For a review on factors involved in NER pathway see de Laat, Jaspers et al. (1999).

Recognition of bulky distortions leads to the removal of a short single-stranded DNA segment, approximately 30 base-pair long, which includes the lesion, creating a single-strand gap in the DNA. Excision occurs exclusively on the DNA strand that contains the DNA adduct: the proteins involved in NER are able to distinguish not only damaged vs. undamaged duplex DNA but also which strand contains the adduct (Shuck, Short et al. 2008). The gap is subsequently filled in by DNA polymerase (often called repaired patches), which uses the undamaged strand as a template. The repaired DNA patches are usually defined as unscheduled DNA synthesis (UDS) in order to distinguish them from normal replicated DNA, which is considered as cellular scheduled activity.

Lack or deregulation in the NER pathway results in severe diseases (Xeroderma Pigmentosum (XP), Cockayne's syndrome and Trichothiodystrophy) and predisposition to cancer development. NER genes have been the subject of intense screening for possible SNPs related to carcinogenesis (Kiyohara and Yoshimasu 2007; Crew, Gammon et al. 2007). The general attempt of the scientific community is to delineate connections between DNA repair capacity and genetic instability that eventually correlate with probability of cancer development. Many of these analyses are contradictory and present a considerable challenge since they are often unable to measure the starting parameter: DNA repair capacity. Recent results on models containing multiple SNPs within the repair pathway have demonstrated greater correlation to cancer risk and response to chemotherapeutics (Bartsch, Dally et al. 2007).

In addition to a role in carcinogenesis, NER capacity is also relevant in the context of cancer treatment. Numerous chemotherapeutic agents, including platinum derivatives (cisplatin, oxaliplatin, carboplatin are the most common) act via the formation of bulky DNA adducts, which are NER targets (Jamieson and Lippard 1999). Individual chemosensitivity is therefore influenced by NER repair capacity and the latter could be used to predict therapy response. Platinum derivatives are the major treatment for a variety of cancers, including testicular, lung and ovarian cancers, as well as tumors of the head and neck (Shuck, Short et al. 2008). The importance of DNA repair capacity is demonstrated by the dramatic discrepancy of cisplatin efficacy in two clinical cases: testicular cancer, which is characterized by strongly reduced DNA repair capacity, shows 95% survival rate after cisplatin treatment (Einhorn 2002) while non-small cell lung cancer (NSCLC), present with higher levels of DNA repair capacity has low survival (Spitz, Wei et al. 2003). Furthermore, NER deficiency seems to play an important role in the etiology of sporadic breast cancer. Thus platinum-derivatives are expected to be effective in the treatment of early-stage breast cancer (Latimer, Johnson et al. 2010) and NER repair capacity could be used to predict therapy response for this type of cancer too. Considering these correlations, precise measurements of NER repair capacity would help increasing the efficacy of current chemotherapeutic agents that work by damaging DNA (Kartalou and Essigmann 2001). In addition, the method of the invention could be used to identify breast epithelium showing reduction in NER capacity, and thus serve as predictive test of tumorigenesis.

NER activity is estimated from UDS measurements. UDS is generally detected by incorporation of modified/labeled nucleosides during cell cultures, similarly to DNA replication. However, due to the small size (about. 30 bp patches) and lower frequency of these events around the genome, it is difficult to quantify UDS with high sensitivity or localize patches around the genome. Measurements must be performed on non-S-phase cells or replication DNA synthesis has to be silenced in order to detect UDS (Lehmann and Stevens 1980), which otherwise will be masked by replication signal. Standard tests in diagnostic laboratories are: ³H-thymidine incorporation, followed by liquid scintillation counting or autoradiography of tissue-culture substrates and evaluation of grain counting; Bromodeoxyuridine (BrdU) incorporation, followed by immune-fluorescent detection by anti-BrdU antibodies (Kelly and Latimer 2005). Radioactivity allows reaching higher sensitivities than immunodetection, but is more labor-intensive and time-consuming. The results are used to diagnosis repair-deficient disorders clinically and provide a basis for investigation of repair deficiency in human tissues or tumors. At the present time, no other functional assay is available that directly measures the capacity to perform NER on the entire genome without the use of radioactivity or specific antibodies.

The inventors have developed methods to detect UDS directly on stretched DNA, in a time- and cost-effective fashion, and with none of the constraints of manipulating radioactivity or the drawbacks of antibodies. Moreover, the method of the invention enables to estimate NER repair capacity of a cellular sample exposed to DNA damaging treatment, whatever the type of treatment considered. With a single-molecule approach like Molecular Combing, UDS detection would gain sensitivity, resolution and mapping studies would be possible thanks to simultaneous hybridization. When modified-nucleosides are provided to cells in culture after UV-exposure, NER will produce small oligopatches (for example, from 20 to 40 bp, and preferably 30 bp) and the replication machinery will produce much longer labeled fragments. As a result, fluorescent signal from UDS will appear like a spotty-signal on stretched DNA molecules while replication will correspond to long linear signals. The approach is the sole allowing simultaneous detection of replication and UDS signals. This would significantly help the discovery of drugs with increased specificity, since candidate compounds potentially perturb several DNA metabolic pathways, including DNA replication and recombination, and broad effects can yield dramatic clinical responses.

The inventors also disclose a method for detecting *in vitro* the presence NER-driven UDS in cells exposed to genotoxic agents, in particular the detection of UDS in human normal fibroblasts exposed to UV light. Said method comprises a step of incubation with alkyne-modified nucleosides during cell culture and chemical detection of NER patches on stretched DNA. Direct visualization of 30 bp fluorescent segments on stretched DNA has never been demonstrated. In our experience, spotty-signals on stretched molecules correspond to several hundreds of bases when using a standard CCD camera. Single-patches could be visualized using a high sensitivity camera. NER patches are probably concentrated in clusters (Svetlova, Solovjeva et al. 2002): clusters covering a region shorter than 1000 bp would appear like intense spots because of optical resolution, while patches spaced more than this resolution could be distinguished as separated smaller spots. Therefore, to perform the detection of UDS, a protocol that does not produce spotty background noise is needed: immunochemistry methods are excluded because antibodies are known to produce background noise in the form of spots. Moreover, a 30 bp sequence should contain in average 6-10 thymidines. This means that only a few labeled nucleotides will be incorporated during repair even in the best conditions. Post-synthetic detection must then convert labeled nucleotides to fluorescence with the highest efficiency. These elements oriented us to the development of a chemical detection method. We adapted a novel post-synthetic fluorescent detection based on Click chemistry: alkyne-labeled uridines (EdU) incorporated into the DNA by cells are converted to fluorescent-nucleotides on combed DNA by specific chemical reaction with azide-labeled fluorophores (Salic and Mitchison 2008).

### Experimental procedures

### Culture of Human Normal Fibroblasts

Experiments were performed using human normal skin fibroblasts GM08402 and HS 707(B) and human XP-C donor skin fibroblasts XP17BE (all from ATCC Cell Bank). Cells from cryogenized stocks were thawed quickly, plated at a density of 10⁴ cells/cm² and cultured to confluence in standard Petri dishes at 37° C, 5% CO₂. Growth medium used was MEM (Modified Eagle Medium, Invitrogen) with 15% v/v FBS (Invitrogen), 2% v/v Glutamine (Gibco, Invitrogen), 2% v/v NEAA (Gibco, Invitrogen). Cells GM08402 were kept at confluence for 1 day and then incubated 6h with MEM containing 0.5% FBS, to further reduce replication. Cells HS 707(B) and XP17BE were harvested at 80% confluence.

### UV light treatment and UDS labeling

Cells in Petri dishes were exposed to UV-C light produced by a germicidal lamp (Philips, 254 nm, 15 W, 0.8 µW/m²), lid open. Exposure doses were set to 150 and 250 J/m² for cell line GM08402 and to 10, 20 and 30 J/m² for cell lines HS 707(B) and XP17BE. Radiation doses were measured with a UV-C radiometer (LT Lutron, ref. Q569239). Control samples were simply exposed to air by opening the Petri dish lid. After exposure, media were replaced by MEM containing 100 µM 5-ethynyl-2'-deoxyuridine (EdU) (Invitrogen) and 0.5 % v/v FBS for GM08402 or 15 % v/v FBS for the other cell lines. Cells were incubated for 60 min at 37° C, 5% CO₂ to allow UDS and residual replication labeling.

### Preparation of embedded DNA plugs from cultured cells

After EdU labeling, cells were rinsed once with 1X PBS 20 (Phosphate-Buffered Saline, Invitrogen) at 4°C and once with 1X PBS 20 at RT. Cells were harvested by 3 minutes incubation with 1 ml commercial Trypsine-EDTA solution (Trypsin 0.05% in 0.53 mM EDTA, Invitrogen). Trypsine digestion was stopped by addition of 9 ml growth medium and cells were counted using disposable 25 counting chambers (Kova slide, CML), centrifuged at 1000 rpm for 5 minutes and resuspended in 1X PBS buffer/Trypsine EDTA, ratio 1:1 to final concentrations of 5x10⁵ to 2x10⁶ cells/mL. Cell suspension was then mixed thoroughly at a 1:1 ratio with a 1.2% w/v solution of low-melting point agarose (Nusieve GTG, ref. 50081, Cambrex) prepared in 1 X PBS at 50°C. 90 µL of the cell / agarose mix was poured in a plug-forming well (BioRad, ref. 170-3713) and left to cool down at least 15 min at 4 °C. Lysis of cells in the blocks was performed as previously described (Schurra and Bensimon 2009). Briefly, Agarose plugs were incubated overnight at 50 °C in 250 µL of a 0.5M EDTA (pH 8), 1 % Sarkosyl, 250 µg/mL proteinase K (Eurobio, code : GEXPRK01, France) solution, then washed three times in a Tris 10mM, EDTA 1 mM solution for 30 min at room temperature.

### Final extraction of DNA and Molecular Combing

Plugs of embedded DNA from human fibroblasts were treated for combing DNA as previously described (Schurra and Bensimon 2009). Briefly, plugs were melted at 68 °C in a MES 0.5 M (pH 5.5) solution for 20 min, and 1.5 units of beta-agarase (New England Biolabs, ref. M0392S, MA, USA) was added and left to incubate for up to 16h at 42° C. The DNA solution was then poured in a Teflon reservoir and Molecular Combing was performed using the Molecular Combing System (Genomic Vision S.A., Paris, France) and Combicoverslips (20 mm x 20 mm, Genomic Vision S.A., Paris, France). The combed surfaces were cured for 4 hours at 60 °C.

### Detection of UDS and Replication Signal

Detection of alkyne-labeled nucleotides was performed by Cu(I)-catalyzed Huisgen cycloaddition (Click) reaction as previously described (Salic and Mitchison 2008). Briefly, a reaction mixture of 100 mM Tris Buffer pH 8.5, 0.5 mM CuSO₄ (Sigma), 1 µM Alexa Fluor® 594 azide (Invitrogen) and 50 mM sodium L-ascorbate (Sigma) (added last to the mix from a 0.5 M solution) was freshly prepared and mixed with Block-Aid (Invitrogen, ref. B-10710) in a 1:1 ratio. 20 µl of reaction mixture were poured on top of clean glass slides and covered with a combed surface. Cover-slips were incubated for 30 min at RT protected from light, then rinsed for 1 min in deionised water with agitation at 500 rpm. A second incubation with a newly prepared reaction mixture was performed for other 30 min. Surfaces were then rinsed twice in Tris 10 mM/EDTA 1mM for 5 minutes and once in deionised water for 1 minute, both with agitation at 500 rpm. Residual water was dried with compressed air.

### Analysis of Fluorescent Signals

For direct visualization of UDS and Replication signals on combed DNA, cover-slips were mounted with 20 µL of protein-based blocking agent (for example, Block-Aid from Invitrogen)-and YOYO-1 iodide (Molecular Probes, code Y3601) mixture (10000:1 v/v) in order to counter-stain all stretched molecules. Imaging was performed with an inverted automated epifluorescence microscope, equipped with a 40X objective (ImageXpress Micro, Molecular Devices, USA). Length of the YOYO-1-stained DNA fibers and of the linear EdU signals were measured and converted to kb using an extension factor of 2 kb/µm (Schurra and Bensimon 2009), with an internal software GVlab v0.4.6 (Genomic Vision S.A., Paris, France).

### Results

### DNA extraction from UV exposed human fibroblasts

Molecular Combing has been successfully performed with DNA solution from isolated cells including cultured cells (i.e., established cell strains, immortalized primary cells) or biological fluids (*i.e*., peripheral blood lymphocytes, amniotic cells) (Gad, Klinger et al. 2002; Caburet, Conti et al. 2005). During standard sample preparation, many DNA molecules are sheared at random location due to uncontrolled manipulation forces resulting in high variability in the size of DNA prepared. It has been shown that molecular weight of combed DNA can be increased when chromatin is embedded and deproteinised in an agarose plug (Lebofsky and Bensimon 2003). With this protocol, the analyzed DNA molecules are of variable length, but the average length is about 200 kb, with longest molecules reaching several megabases. The length distribution of the molecules is also affected by the quality of the DNA: when cells are exposed to UV, SSB are generated, weakening the mechanical resistance of the molecules and increasing the frequency of molecule breakage. Since Molecular Combing on UV-exposed fibroblasts has never been performed, we analyzed the effect of UV on DNA length distribution. The results are plotted in Figure 1: the damage induced by UV exposure creates "fragile sites" in a dose-dependent manner. The curves show clearly the effect of radiation on DNA mechanical resistance: when UV dose increases (from 150 to 250 J/m²), the length of combed DNA molecules is progressively reduced.

### Chemical detection of signals on combed DNA

Chemical detection, and in particular Huisgen cycloaddition of alkynes and azides (Click chemistry), has recently been added to the repertoire of DNA labeling methods, showing exceptional detection efficiency comparing to immunochemistry techniques (Gierlich, Burley et al. 2006). The main advantage of Click reaction is its bio-orthogonality, because reactive groups involved are generally absent in biological materials. Chemical detection of functionalized nucleotides on stretched and more generally substrate-immobilized DNA has never been reported before. The method has been successfully employed to study DNA replication (Salic and Mitchison 2008) and UDS (Limsirichaikul, Niimi et al. 2009) in fixed cellular samples and tissue. However, the reaction conditions optimized for such types of samples are not appropriate in the situation of biological material immobilized on an inorganic substrate, where reaction orthogonality is reduced. We tested different parameters, including dye concentration and repeated incubations to increase final EdU to (Alexa Fluor®)dU conversion efficiency and reduce background and spotty noise to minimum. An example of the results of best parameters combination is shown in Figure 2: replication signal on combed DNA is detected subsequently with anti-BrdU antibodies (green) and chemical detection (red). Chemical detection does not require denaturation of DNA and produces a continuous signal, while fragmented detection is observed with antibodies. Moreover, noisy spots are observed in green, due to non-specific adsorption of antibodies, but no red spots are present: the red background level is quite intense but uniform, which allows reliable detection of small events on the molecules like UDS.

### Visualization of UDS and estimation of NERCA at high UV doses

UDS is usually detected in fixed cellular samples by measuring nuclear fluorescence intensity of selected non-dividing cells. Measurements are performed comparing the global level of fluorescence intensity of sampled nuclei to a reference sample. Visualization of distinguished NER activity in the nucleus of fixed quiescent fibroblasts has been reported by a single group, who demonstrated for the first time direct detection of clustered UDS and their global positioning in the nucleus (Svetlova, Solovjeva et al. 2002). In contrast to immunochemistry assays that have reduced resolution, with single molecule approaches like Molecular Combing it is possible to detect NER patches or estimate the number or the distance of repair events present in a cluster.

The results obtained were first analyzed by exposing quiescent fibroblasts GM08402 to high (>50 J/m²) UV doses. Two types of signals on YOYO-1 stained combed molecules were observed: spotty and linear signals (Figure 3). The same number of images was analyzed per sample. From molecule length measurements, we deduced the total quantity of genetic material (in Mbp) present on the analyzed regions of the slides. This parameter is needed to estimate Nucleotide-Excision Repair Capacity (NERCA) starting from UDS signal detection. The NERCA can be expressed as the ratio between the number of UDS signals detected in a region R and the total amount of genetic material stretched on the region R, which represents a normalized value. When the regions analyzed are sufficiently large (several genomes analyzed), the NERCA values obtained from different slides and conditions can be compared to extrapolate significant differences. Image analysis of GM08402 is summarized in Table 2. On the control samples (non-exposed to UV light), 150 linear and 6 spotty signals were observed; on the 150 J/m² UV-exposed sample, 55 spotty and 13 linear signals; on the 250 J/m² UV-exposed samples, 149 spotty and 37 linear signals. Linear signals were attributed to DNA replication (6h quiescence were not sufficient to silent replication completely). The ratio of spotty/linear signals indicates that when UV-exposure is performed, much more spotty-signals appear. The major part of these spotty signals corresponds then to UDS performed by the NER system The analysis of several thousands of Mbp of DNA indicated that the number of UDS sites increases exponentially with the applied dose of UV-C (Figure 4, *A*)*,* as confirmed by the linear decrease of their average distance on the combed DNA molecules (Figure 4, *B).* For the GM08402 cellular sample and the level of genotoxic exposure studied, NER Capacity (NERCA) appears to be constant up to the dose of UV-C tested. If the NERCA had varied, a deviation from the linear profile of UDS distance should be observed.

**Table 2: Frequency of signals observed on combed DNA extracted from partially quiescent GM08402 normal human fibroblasts exposed to different doses of UV-C light. The number of spotty signals, associated to UDS, increases with the UV-C dose, as attested by the linear decrease of their average distance on the combed DNA molecules.**

| UV-C Dose 254 nm (J/m²) | Number of Spotty Signals | Number of Linear Signals | DNA analyzed (Mbp) | Average Distance of Events (Mbp) |
|---|---|---|---|---|
| 0 | 6 | 150 | 987 | 164.5 |
| 150 | 55 | 13 | 4094 | 74.4 |
| 250 | 149 | 37 | 2785 | 18.7 |

### Visualization of UDS and estimation of NERCA at physiological UV doses

We then compared the results obtained by exposing normal, repair-proficient HS 707(B) and repair-deficient XP17BE fibroblasts to incremental doses of UV-C in the range considered physiologically relevant. A total of 8 cellular samples were prepared and analyzed, to provide a comparative base for the repair capacity at 4 different UV-C doses: no UV exposure (0 J/m²), 10 J/m², 20 J/m² and 30 J/m² UV doses. The accuracy of the results was ensured by experimental planning and data were accumulated until at least 30 UDS signals were counted for each condition, with the exception of sample XP-C 10 J/m². The size and the intensity of the fluorescent UDS spots observed is not constant: some signals show larger spreading and higher intensity. The method of the invention has indeed the potential to discriminate the parameter "number of repair patches" from the parameter "size of the patches". Thus, we proceeded first with the quantification of the frequency of events per each condition and afterwards we reanalyzed the results to investigate the fluorescence intensity of the UDS signals.

The analysis of the frequency of repair events for the 8 conditions studied is summarized in Table 3 and represented in Figure 5. We employed the ratio 'number of UDS signals'/'amount of DNA analyzed' as estimator for the probability of observing a UDS event. The Poisson distribution is a convenient approximation to model the probability of UDS events. The parameter λ characterizing the distribution is the number of observations of the event, i.e. the number of detected UDS signals. Poisson confidence limits are provided in the table and illustrated in the graph of Figure 5 in the form of error bars.

The evolution of the probability P(UDS) with increasing UV-dose shows clearly the deficient repair capacity of the XP-C cellular sample. At 0 dose, practically the same baseline frequency of UDS signals was observed in both samples. After UV-exposure, a significant number of UDS signals were detected for normal fibroblasts (HS 707(B)), while only a slight increase in repair synthesis was observed for the XP-C sample (XP17BE). It is important to notice that the trend of P(UDS) in Fig. 5 changes after 20 J/m² for the repair-proficient sample. The frequency of events tends to a plateau. This finding is consistent with previous observations made with both direct and indirect techniques. After a critical UV-dose of 20 J/m², the NER system saturates and the repair synthesis proceeds at constant yield (Ahmed and Setlow 1977).

The expected repair capacity of the XP-C cell line is comprised between 30 and 60% of the control, according to the UDS measurements carried out by the cell provider. If we calculate the direct ratio P(UDS) of XP-C cells/ P(UDS) Normal cells we find ∼57% residual XP-C repair capacity for the 20 J/m² condition and ∼64% for the 30 J/m² condition. The values found are also consistent with the range designated by the cell provider and further confirm the reliability of the method of the invention.

The direct observation of repair synthesis on single DNA molecules allows drawing a much more informative picture of the repair activity. As we mentioned, UDS spots vary in size and fluorescence intensity. We proceeded thus to the analysis of the "fluorescence content" of the signals, which constitutes an index of the amount of repair synthesis performed in each site. We employed a simple method to roughly but equitably quantify relative fluorescence variations of spots belonging to a same substrate. Pixel intensities were quantified and then an internal normalization was applied for all signals within a substrate. We obtained 8 groups of "fluorescence content" data (one per sample) that are plotted in the form of histograms in Figure 6.

**Table 3: Estimation of the probability P(UDS) of observing a UDS event on combed DNA extracted from normal human fibroblasts HS 707(B) and NER-deficient fibroblasts XP17BE exposed to 4 doses of UV-C light. The occurrence of a UDS event was modeled as a Poisson variable.**

| UV dose (J/m²) | Cell Line | DNA analyzed (*N* Mbp) | UDS events (λ) | P (UDS) (*p*) | Confidence Interval P(UDS) | |
|---|---|---|---|---|---|---|
| 0 | Normal Fibroblasts | 9503.43 | 36 | 0.00379 | 0.00290 | 0.00488 |
| | XP-C Fibroblasts | 15962.53 | 63 | 0.00395 | 0.00322 | 0.00480 |
| 10 | Normal Fibroblasts | 21555.36 | 131 | 0.00608 | 0.00527 | 0.00698 |
| | XP-C Fibroblasts | 4464.32 | 18 | 0.00403 | 0.00270 | 0.00558 |
| 20 | Normal Fibroblasts | 7166.16 | 56 | 0.00781 | 0.00624 | 0.00953 |
| | XP-C Fibroblasts | 17170.24 | 79 | 0.00460 | 0.00381 | 0.00545 |
| 30 | Normal Fibroblasts | 11639.94 | 96 | 0.00825 | 0.00695 | 0.00963 |
| | XP-C Fibroblasts | 18672.59 | 100 | 0.00536 | 0.00453 | 0.00624 |

A clear trend appears from the histograms: the distribution of the normalized intensities shifts to higher values when the UV-dose increases. In the control non- irradiated samples, data are distributed with good symmetry around a clear peak. After exposure to increasing doses of radiation, the distribution of the data deviates progressively from the Gaussian model. Particularly, the peak positioned at relative fluorescence content equal to 2 drops, since more signals have larger fluorescence content. The logical interpretation is that some repair patches are widening at certain sites on the DNA molecule. The shift is far more pronounced for normal fibroblasts than the XP-C cells, though an effect is visible for both cell types. For the first time, an assay provides an insight on the reorganization of the NER pathway in response to critical doses of damage. The saturation in the frequency of UDS is detected as reported in previous art, but the single-molecule approach enables discriminating two contributions from the bulk of repair synthesis: the number of sites saturates, but the size of some repair patches continues to grow. These findings highlight the great added-value of the method of the invention to unravel the complex relationship linking DNA damage to mutagenesis.

### A variant method

The detection and global quantification of Unscheduled DNA Synthesis can be performed similarly to the method described in the previous paragraph using single DNA molecules immobilized on a substrate in a non-stretched configuration, for instance adsorbed in random coil form or only partially elongated. In the absence of uniform and constant DNA stretching, the quantification of the total amount of DNA relies on the measurement of the fluorescence intensity of the DNA-binding dye employed (in the example, YOYO-1). Similarly, the distinction between replication DNA synthesis and UDS can be based on the level of intensity of the EdU signal: under a definite intensity, which can be absolute or related to the intensity of the DNA-binding dye, the signal is considered UDS and over a second level of intensity, the signal is associated to replication. This type of detection on single, immobilized DNA molecules has never been reported before and offers a dramatic improvement in resolution and sensitivity with respect to standard methods that use immobilized cells or chromosomes on substrates.

### Example 3

### Example 3A: Detection of lesion or repair by a detectable element that specifically binds to the target.

### 2.1 Direct visualization of SSB and DSB

Personalized radiation therapy holds the promise that the diagnosis, prevention, and treatment of cancer will be based on individual assessment of risk. Although advances in personalized radiation therapy have been achieved, the biological parameters that define individual radiosensitivity remain unclear. Predicting normal tissue and tumor radiosensitivity has been the subject of intensive investigation, but has yet to be routinely integrated into radiotherapy (Torres-Roca and Stevens 2008). Many predictive factors of tumor radiosensitivity have been described. Number of clonogenic cells, proliferation rate, hypoxia and intrinsic radiosensitivity are usually considered as the main parameters of tumor control (Hennequin, Quero et al. 2008). Complication risks for an individual irradiated patient can be predicted currently only by the complication rates seen in similar populations. This assessment fails to account for variation in the DNA repair capacity of the individual. Moreover, predicting tumor radiosensitivity has significant clinical applicability. If such prediction could be done accurately, radiation doses could be tailored to the radiocurability of individual tumors. In addition, such an assay could be helpful in determining the optimal doses and schedules of biological and chemotherapeutic radiosensitizers. Several groups have published modeling data demonstrating the clinical value of predicting normal tissue and tumor radiosensitivity (Mackay and Henry 1999; MacKay, Niemierko et al. 1998). These data indicate that both probabilities of tumor control and normal tissue complication can potentially be improved by individualizing treatment according to the results of predictive assays.

Radiotherapy kills cells primarily through extensive DNA damage. Ionizing radiation carries a lot of energy which is able to induce a large amount of breaks in the DNA backbone. When single-strand breaks (SSB) are closer than a critical distance on the molecule, they form a double-strand break (DSB), which represents the most dangerous lesion for the cell and the cytotoxic effect associated to radiation (Suzuki, Ojima et al. 2003). Intrinsic radiosensitivity is correlated to the global ability of the cell to detect and repair DNA damage, but more particularly to the capacity of repairing DSBs. There have been numerous attempts to extrapolate a biomarker for radiosensitivity based on the evaluation of DSB repair capacity and kinetics. Single-cell gel electrophoresis (comet assay) has been used to study rejoining kinetics of DSB and radio-resistant hypoxic cells in solid tumors and tissues (P.L. Olive 2009). Ismail et al. developed an assay that analyzes DSB through DNA end-binding complexes. The assay showed to predict radiosensitivity in both primary fibroblasts and cancer cell lines (Ismail, Puppi et al. 2004). Recent immunohistochemical techniques allowed reaching high sensitivity in the detection of DSB (Vasireddy, Sprung et al. 2010), but they have not been adopted in clinical routine because of their experimental complexity. Microaway-based gene expression profiling has importantly contributed to the understanding of the relationship between intrinsic radiosensitivity and clinical outcome, enabling to differentiate between patients with a high and low risk of radiation-induced fibrosis (Fernet and Hall 2008). However, the technical set-up for gene expression measurements means that this latter assay is unlikely to be introduced soon into a routine clinical setting.

Despite more than a decade of research efforts into predictive radiation oncology, none of these assays has met the requirements of clinical applicability. The chromosomal fragments generated by DSB carry significant information regarding frequency of strand breakages, physical and spatial location of these events and relationship with the level of exposure to radiation. However, they are rare events (60-200 events per genome at standard doses of ionizing radiation) and there is a need for a sensitive technique that provides an accurate analysis of their amount and distribution.

The inventors have found that a labeling approach is successfully employed to directly detect and quantify breakages on the backbone of stretched DNA molecules. Every free-end of a broken strand (SSB or DSB) exposes a 3' and a 5' extremity. The inventors reasoned that, if all free 3' ends are labeled just after cell exposure to a genotoxic agent, it is possible to visualize them directly on single DNA molecules and quantify them precisely. The inventors expected to detect SSB as fluorescent spots in the middle of a combed molecule and DSB at the extremity. In order to label 3' free-ends, we used a powerful enzyme called Terminal Deoxynucleotide Transferase (TdT), which is able to elongate 3'-OH free-ends with several hundreds of nucleotides and without template DNA. This approach was tested on lambda phage DNA in order to check if the labeled oligos polymerized by TdT on the nicks and the extremities could be detected.

### Experimental Procedures

### Preparation of Lambda phage DNA Solution and Molecular Combing

The tailing reaction mixture (50 µl) was prepared by mixing 1mM EdUTP (Invitrogen), 10 pM Lambda phage DNA (Sigma) and 15 U of TdT enzyme (Invitrogen) in 1X TdT Reaction Buffer (Invitrogen) and was incubated at 37°C for 3h. The reaction was stopped by adding 0.1 M EDTA to the mixture. The DNA combing solution was prepared by diluting the mixture into 2 ml of MES buffer 0.5 M (pH 5.5). The solution was poured in a Teflon reservoir and Molecular Combing was performed using the Molecular Combing System (Genomic Vision S.A., Paris, France) and Combicoverslips (20 mm x 20 mm, Genomic Vision S.A., Paris, France). The combed surfaces were cured for 4 hours at 60 °C.

### Detection of EdU tails

Detection of alkyne-labeled tails was performed by Cu(I)-catalyzed Huisgen cycloaddition (Click) reaction as previously described (Salic and Mitchison 2008). Briefly, a reaction mixture of 100 mM Tris Buffer pH 8.5, 0.5 mM CuSO₄ (Sigma), 1 µM Alexa Fluor® 594 azide (Invitrogen) and 50 mM sodium L-ascorbate (Sigma) (added last to the mix from a 0.5 M solution) was freshly prepared and mixed with Block-Aid (Invitrogen), ref. B-10710) in a 1:1 ratio. 20 µl of reaction mixture were poured on top of clean glass slides and covered with a combed surface. Cover-slips were incubated for 30 min at RT protected from light, then rinsed for 1 min in deionised water with agitation at 500 rpm. A second incubation with a newly prepared reaction mixture was performed for other 30 min. Surfaces were then rinsed twice in Tris 10 mM/EDTA 1mM for 5 minutes and once in deionised water for 1 minute, both with agitation at 500 rpm. Residual water was dried with compressed air.

### Analysis of Fluorescent Signals

For direct visualization of signals on combed DNA, cover-slips were mounted with 20 µL of a Block-Aid (Invitrogen, ref. B-10710)-YOYO-1 iodide (Molecular Probes, code Y3601) mixture (10000:1 v/v) in order to counter-stain all stretched molecules. Imaging was performed with an inverted automated epifluorescence microscope, equipped with a 40X objective (ImageXpress Micro, Molecular Devices, USA).

### Results

The results of the proof of concept on lambda DNA show that end-labeling is more than 60% efficient and that nicks (SSB) are visible in the middle of combed molecules as expected (Figure 7).

From the duration of tailing reaction and the amount of EdUTP present in the mixture, we calculated that tails length is comprised between 150 and 200 nucleotides, which is sufficient to detect an intense red signal on YOYO-1 labeled DNA.

End-labeling efficiency has been calculated as the ratio of labeled and unlabeled extremities on a sample constituted of 2000 stretched lambda DNA molecules. The efficiency of the proof of concept already exceeds 60% and can be certainly increased with some simple optimization.

Due to its direct and high-throughput nature, this method allows precise quantification of chromosomal DNA fragmentation and can be applied to small amounts of starting material, including blood and tissue biopsy, in a time- and cost-effective fashion. Together, these features make it suitable for the requirements of clinical applicability and render it a powerful tool to understand the biological parameters influencing individual radiosensitivity. Moreover, the method of the invention has great potential for general biomonitoring studies, beyond SSB and DSB quantification: using DNA hybridization on combed molecules, chromosomal localization of DNA damage and detection of chromosomal aberrations can be coupled in a single test.

### A variant method

The detection and global quantification of SSB/DSB can be performed similarly to the method described in the previous paragraph using single DNA molecules immobilized on a substrate in a non-stretched configuration, for instance adsorbed in random coil form or only partially elongated. In the absence of uniform and constant DNA stretching, the quantification of the total amount of DNA can rely on the measurement of the fluorescence intensity of the DNA-binding dye employed (in the example, YOYO-1). Similarly, the amount of breaks (SSB and DSB) can be derived from the quantification of the intensity of the EdU signal, in a relative manner with respect to an internal reference or in a more absolute way by taking into account the average tail-length, the efficiency of the end-labeling and the conversion efficiency from EdU to fluorescence.

### Example 3B: 2.2 Direct visualization and localization of damage by chemical reaction.

Detection and localization of abasic sites (AP sites) with Aldehyde Reactive Probes on a Selected Gene hybridized on stretched DNA.

### Experimental procedures

### Culture of cell line

The method of culture used is similar to the methods described above for human normal fibroblasts in the case of adherent cells and for human normal lymphoblasts in the case of suspension cells.

### ARP (aldehyde reactive probe) treatment

For the *in vivo* ARP labeling, cells were incubated for 60 min with the aldehyde reactive probes (Cayman Chemical) tagged with biotin or a fluorochrome at 37° C, 5% CO₂ to allow probes react with the ring-open form of AP sites to generate a biotin-tagged or fluorescently-labeled AP site.

### Preparation of embedded DNA plugs from cultured cells

After ARP labeling, cells were rinsed once with 1X PBS 20 (Phosphate-Buffered Saline, Invitrogen) at 4°C and once with 1X PBS 20 at RT. Cells were harvested by 3 minutes incubation with 1 ml commercial Trypsine-EDTA solution (Trypsin 0.05% in 0.53 mM EDTA, Invitrogen). Trypsine digestion was stopped by addition of 9 ml growth medium and cells were counted using disposable 25 counting chambers (Kova slide, CML), centrifuged at 1000 rpm for 5 minutes and resuspended in 1X PBS buffer/Trypsine EDTA, ratio 1:1 to final concentrations of 5x10⁵ to 2x10⁶ cells/mL. Cell suspension was then mixed thoroughly at a 1:1 ratio with a 1.2% w/v solution of low-melting point agarose (Nusieve GTG, ref. 50081, Cambrex) prepared in 1 X PBS at 50°C. 90 µL of the cell / agarose mix was poured in a plug-forming well (BioRad, ref. 170-3713) and left to cool down at least 15 min at 4 °C. Lysis of cells in the blocks was performed as previously described (Schurra and Bensimon 2009). Briefly, Agarose plugs were incubated overnight at 50 °C in 250 µL of a 0.5M EDTA (pH 8), 1 % Sarkosyl, 250 µg/mL proteinase K (Eurobio, code : GEXPRK01, France) solution, then washed three times in a Tris 10mM, EDTA 1 mM solution for 30 min at room temperature.

### Final extraction of DNA and Molecular Combing

Plugs of embedded DNA from human fibroblasts were treated for combing DNA as previously described (Schurra and Bensimon 2009). Briefly, plugs were melted at 68 °C in a MES 0.5 M (pH 5.5) solution for 20 min, and 1.5 units of beta-agarase (New England Biolabs, ref. M0392S, MA, USA) was added and left to incubate for up to 16h at 42° C. The DNA solution was then poured in a Teflon reservoir and Molecular Combing was performed using the Molecular Combing System (Genomic Vision S.A., Paris, France) and Combicoverslips (20 mm x 20 mm, Genomic Vision S.A., Paris, France). The combed surfaces were cured for 4 hours at 60 °C.

### Synthesis and labeling of probes to localize the Selected Gene on stretched DNA

Probe size ranges from 100 to 3000 bp in this example. The specific probes for the Selected Gene were produced by long-range PCR using LR Taq DNA polymerase (Roche) using the appropriate primers and commercial human DNA as template DNA. PCR products were ligated in the pCR®2.1 vector using the TOPO® TA cloning Kit (Invitrogen, France, code K455040). The two extremities of each probe were sequenced for verification purpose. The apparent probes of 4 different sizes in this example are mixes of several adjacent or overlapping probes. Labeling of the probes with 11-digoxygenin-dUTP was performed using conventional random priming protocols. The reaction products were visualized on an agarose gel to verify the synthesis of DNA.

### ARP (aldehyde reactive probe) treatment

ARP reaction can be performed during cell culture prior to DNA extraction or after DNA stretching on the combed slide. DNA combed slides were incubated for 30 min with the aldehyde reactive probes (Cayman Chemical) tagged with biotin or a fluorochrome at 37° C, to allow probes react with the ring-open form of AP sites to generate a biotin-tagged or fluorescently-labeled AP site.

### Hybridization of probes to localize the Selected Gene

Subsequent steps were also performed essentially as previously described in Schurra and Bensimon, 2009 (Schurra and Bensimon 2009). Briefly, a mix of labeled probes (250 ng of each probe) were ethanol-precipitated together with 10µg herring sperm DNA and 2,5µg Human Cot-1 DNA (Invitrogen, ref. 15279-011, CA, USA), resuspended in 20 µL of hybridization buffer (50 % formamide, 2X SSC, 0.5 % SDS, 0.5 % Sarcosyl, 10mM NaCl, 30 % Block-aid (Invitrogen, ref. B-10710, CA, USA). The probe solution and the stretched DNA were heat-denatured together on the Hybridizer (Dako, ref. S2451) at 90 °C for 5 min and hybridization was left to proceed on the Hybridizer overnight at 37 °C. Slides were washed 3 times in 50 % formamide, 2x SSC and 3 times in 2x SSC solutions, for 5 min at room temperature.

### Detection of labeled AP sites and hybridized probes

AP sites treated with fluorescently labeled ARP could be directly visualized with an epi-fluorescence microscope. Biotin-tagged AP site were detected using fluorescently-labeled streptavidin (Invitrogen). Surfaces were then rinsed twice in Tris 10 mM/EDTA 1mM for 5 minutes and once in deionised water for 1 minute, both with agitation at 500 rpm. Residual water was dried with compressed air. Probes detection was performed using antibody layers. For each layer, 20 µL of the antibody solution was added on the slide and covered with a combed coverslip and the slide was incubated in humid atmosphere at 37 °C for 20 min. The slides were washed 3 times in a 2x SSC, 1 % Tween20 solution for 3 min at room temperature between each layer and after the last layer. Detection was carried out in this example using a fluorescence-coupled mouse anti digoxygenin (Jackson Immunoresearch, France) antibody in a 1:25 dilution. As second layer, a fluorescence-coupled goat anti mouse (Invitrogen, France) diluted at 1:25 was used. After the last washing steps, all glass cover slips were dehydrated in ethanol and air dried.

### Analysis of Fluorescent Signals

For direct visualization of stretched DNA, cover-slips were mounted with 20 µL of a Block-Aid (Invitrogen, ref. B-10710)-YOYO-1 iodide (Molecular Probes, code Y3601) mixture (10000:1 v/v) in order to counter-stain all stretched molecules. Imaging was performed with an inverted automated epifluorescence microscope, equipped with a 40X objective (ImageXpress Micro, Molecular Devices, USA). Length of the YOYO-1-stained DNA fibers and length of linear signals of hybridized probes and of distances between AP sites were measured and converted to kb using an extension factor of 2 kb/µm (Schurra and Bensimon 2009), with an internal software GVlab v0.4.6 (Genomic Vision S.A., Paris, France).

### Example 3C: 2.3 Direct visualization and quantification of damage by immunofluorescence.

Possible Antibodies: Anti Cyclobutane Pyrimidine Dimers (CPDs), Anti (6-4) photoproducts (6-4 PPs), Anti Dewar photoproducts (Dewar PPs), Anti 8-OH-dG, 8-oxo-G and similar oxidation products, Anti BPDE (Benzo(a)Pyrene DiolEpoxide) DNA adducts, and any future antibody that specifically binds to alterations of the DNA molecule.

### Experimental procedures

### Culture of cell line

The method of culture used is similar to the methods described above for human normal fibroblasts in the case of adherent cells and for human normal lymphoblasts in the case of suspension cells.

### Preparation of embedded DNA plugs from cultured cells

Cells were rinsed once with 1X PBS 20 (Phosphate-Buffered Saline, Invitrogen) at 4°C and once with 1X PBS 20 at RT. Cells were harvested by 3 minutes incubation with 1 ml commercial Trypsine-EDTA solution (Trypsin 0.05% in 0.53 mM EDTA, Invitrogen). Trypsine digestion was stopped by addition of 9 ml growth medium and cells were counted using disposable 25 counting chambers (Kova slide, CML), centrifuged at 1000 rpm for 5 minutes and resuspended in 1X PBS buffer/Trypsine EDTA, ratio 1:1 to final concentrations of 5x10⁵ to 2x10⁶ cells/mL. Cell suspension was then mixed thoroughly at a 1:1 ratio with a 1.2% w/v solution of low-melting point agarose (Nusieve GTG, ref. 50081, Cambrex) prepared in 1 X PBS at 50°C. 90 µL of the cell / agarose mix was poured in a plug-forming well (BioRad, ref. 170-3713) and left to cool down at least 15 min at 4 °C. Lysis of cells in the blocks was performed as previously described (Schurra and Bensimon 2009). Briefly, Agarose plugs were incubated overnight at 50 °C in 250 µL of a 0.5M EDTA (pH 8), 1 % Sarkosyl, 250 µg/mL proteinase K (Eurobio, code : GEXPRK01, France) solution, then washed three times in a Tris 10mM, EDTA 1 mM solution for 30 min at room temperature.

### Final extraction of DNA and Molecular Combing

Plugs of embedded DNA from human fibroblasts were treated for combing DNA as previously described (Schurra and Bensimon 2009). Briefly, plugs were melted at 68 °C in a MES 0.5 M (pH 5.5) solution for 20 min, and 1.5 units of beta-agarase (New England Biolabs, ref. M0392S, MA, USA) was added and left to incubate for up to 16h at 42° C. The DNA solution was then poured in a Teflon reservoir and Molecular Combing was performed using the Molecular Combing System (Genomic Vision S.A., Paris, France) and Combicoverslips (20 mm x 20 mm, Genomic Vision S.A., Paris, France). The combed surfaces were cured for 4 hours at 60 °C.

### Detection of 8-OH-dG and 8-oxo-G oxidation products on stretched DNA

8-hydroxy-2-deoxyGuanosine (8-OH-dG) and 8-oxo-Guanine (8-oxo-G) are products of oxidative damage of DNA by reactive oxygen and nitrogen species and serve as established markers of oxidative stress. Detection was performed using antibody layers. For each layer, 20 µL of the antibody solution was added on the slide and covered with a combed coverslip and the slide was incubated in humid atmosphere at 37 °C for 20 min. The slides were washed 3 times in a 2x SSC, 1 % Tween20 solution for 3 min at room temperature between each layer and after the last layer. Detection was carried out in this example using mouse anti-8-OH-G and mouse Anti-8-oxoG monoclonal antibodies (Abcam) in a 1:25 dilution. As second layer, a fluorescence-coupled goat anti mouse (Invitrogen, France) diluted at 1:25 was used. After the last washing steps, all glass cover slips were dehydrated in ethanol and air dried.

### Analysis of Fluorescent Signals

For direct visualization of stretched DNA, cover-slips were mounted with 20 µL of a Block-Aid (Invitrogen, ref. B-10710)-YOYO-1 iodide (Molecular Probes, code Y3601) mixture (10000:1 v/v) in order to counter-stain all stretched molecules. Imaging was performed with an inverted automated epifluorescence microscope, equipped with a 40X objective (ImageXpress Micro, Molecular Devices, USA). Length of the YOYO-1-stained DNA fibers and length of distances between detected 8-OH-dG and 8-oxo-G sites were measured and converted to kb using an extension factor of 2 kb/µm (Schurra and Bensimon 2009), with an internal software GVlab v0.4.6 (Genomic Vision S.A., Paris, France).

### A variant method

The detection and global quantification of damage using specific antibodies can be performed similarly to the method described in the previous paragraph using single DNA molecules immobilized on a substrate in a non-stretched configuration, for instance adsorbed in random coil form or only partially elongated. In the absence of uniform and constant DNA stretching, the quantification of the total amount of DNA relies on the measurement of the fluorescence intensity of the DNA-binding dye employed (in the example, YOYO-1). The relative amount of damage can be estimated with respect to an internal reference from the quantification of the fluorescence signal associated to the labeled antibodies

### Example 4: Indirect detection and quantification of lesion or repair by converting the targets into molecular extremities

### Profiling Damage by Relative Fragmentation-induced Profile Deviation (PD)

Construction of a DNA length reference profile of a healthy cellular population and comparison of the profile obtained from the same population after exposure to a genotoxic agent is performed. High resolution DNA length reference profiles are constructed by measuring the size of thousands of DNA molecules uniformly stretched on a combed slide.

In the case of damage generated at high frequency along the genome (for instance SSB, abasic sites, methylated, alkylated, oxidated bases, photoproducts), the conversion of most of the targeted lesions into DSB generates theoretically as much new DNA extremities as much lesions were present on the molecules. When comparing the length distribution profile of the damaged sample with the undamaged reference, a Profile Deviation (PD) associated to a specific damage appears. The amount of lesions generated by the genotoxic compound can then be estimated directly from the PD.

The conversion of a specific lesion into a molecular extremity can be performed via enzymatic (Collins, Dobson et al. 1997), chemical or heat treatment (Singh, McCoy et al. 1988). The most reliable method is the use of lesion-specific nucleases, which convert specific types of damage into a SSB or a DSB. If the conversion step generates SSB, a second enzymatic or chemical step can be performed to generate DSB. Common enzymes that can be employed and their targets are summarized in Table 3. By using a selected set of damage-targeting enzymes or treatments, the method of the invention allows studying more than one type of lesion at the same time.

**Table 3: Common enzymes employed for the conversion of specific DNA lesions into SSB or DSB and respective targets. ¹Formamidopyrimidine-DNA glycosylase, ²Human 8-hydroxyguanine DNA-glycosylase, ³T4 pyrimidine dimer glycosylase, ⁴Chlorella Virus Pyrimidine Dimer Glycosylase, ⁵Ultraviolet DNA Endonuclease.**

| Enzyme | Damage Recognized |
|---|---|
| Fpg¹ | 8-oxoguanine, DNA containing formamidopyrimidine moieties |
| Endonuclease III | Thymine glycol, 5,6-dihydrothymine, urea, 5-hydroxy-6-hydrothymine, 5,6-dihydrouracil, alloxan, 5-hydroxy-6-hydrouracil, uracil glycol, 5-hydroxy-5-methylhydantoin, 5-hydroxycytosine, 5-hydroxyuracil, methyltartonylurea, thymine ring saturated or fragmentation product |
| hOGG1² | 8-oxoguanine, DNA containing formamidopyrimidine moieties |
| T4-PDG³ | Cis-syn isomers of cyclobutane pyrimidine dimers |
| cv-PDG⁴ | Cis-syn and trans-syn isomers of cyclobutane pyrimidine dimers |
| UVDE⁵ | Cyclobutane pyrimidine dimers, (6-4) photoproducts |

The inventors have recognized that Molecular Combing, allowing high resolution sizing of dense arrays of uniformly stretched DNA fragments, is successfully applied to the indirect quantification of most DNA lesions. Unlike Molecular Combing, sizing methods based on SCGE (FRAME™, Trevigen; WO1996040902) or single-molecules flowing into micro- (Filippova, Monteleone et al. 2003) or nano-channels (Tegenfeldt, Prinz et al. 2004) do not allow post-processing of the DNA molecules and hybridization studies are very difficult to perform. Moreover, these methods do not provide or provide only partial elongation of DNA molecules. As a consequence, for these methods DNA fragments length has to be estimated from fluorescence intensity measurements, which reduces resolution and measurement precision comparing to DNA stretching techniques. Nothing anticipates the possibility to study DNA damage amount and distribution by the mean of nucleic acid stretching. Furthermore, nothing suggested the application of DNA hybridization on elongated or stretched DNA to investigate the distribution of damage and repair with respect to genome sequence or chromatin organization.

In the following non-limiting example the method Relative Fragmentation-induced Profile Deviation (PD) is successfully applied to the quantification of oxidative damage induced by H₂0₂ exposure.

### Experimental procedures

### Culture of cell line

The method of culture used is similar to the methods described above for human normal fibroblasts in the case of adherent cells and for human normal lymphoblasts in the case of suspension cells.

### H₂O₂ treatment

Cells were incubated for 10 min in growth medium containing 0 (control sample) 1, 5, 10 and 20 mM H₂O₂ at 37° C, 5% CO₂ to induce different doses of oxidative damage.

### Extraction of DNA and Fpg treatment

After H₂O₂ treatment, cells in the 5 samples were harvested and resuspended in 1X PBS 20 (Phosphate-Buffered Saline, Invitrogen) at a concentration of 10⁶ cells/ml. Extraction was performed using PreAnalytiX blood DNA extraction kit (Qiagen) with a custom procedure. Briefly, 1 ml cell suspension per condition was poured in 10 ml BG1 (within PreAnalytiX kit)buffer followed by centrifugation for 5 min at 2500 g. After removal of the supernatant, the pellet was resuspended and rinsed by 5 s mixing in BG2 (within PreAnalytiX kit) buffer. Tubes were spun again for 3 min at 2500 g. The pellets were resuspended in 100 µl Fpg digestion buffer as indicated by the provider (New England Biolabs) and incubated with 3 U of Fpg enzyme (New England Biolabs) for 3h at 37°C. After the digestion, 1 ml BG3 buffer (within PreAnalytiX kit) containing 250 µg/mL proteinase K (Eurobio) was added to the solutions and the tubes were incubated at 65°C for 15 min to allow proteolysis. Afterwards, DNA was precipitated by adding 2-propanol to the tubes in v/v ratio 1:1 and inverting the tubes 20 times. Tubes were incubated overnight at 4°C before removing the supernatant and re-suspending the DNA pellet into 100 µl Tris 40 mM/EDTA 2 mM buffer for a few hours at room temperature.

### Final extraction of DNA and Molecular Combing

Before performing Molecular Combing, 3 ml MES buffer 0.5 M (pH 5.5) were added to each DNA sample and tubes were inverted gently a few times. The DNA solution was then poured in a Teflon reservoir and Molecular Combing was performed using the Molecular Combing System (Genomic Vision S.A., Paris, France) and Combicoverslips (20 mm x 20 mm, Genomic Vision S.A., Paris, France). The combed surfaces were cured for 4 hours at 60 °C.

### Analysis of Fluorescent Signals

For direct visualization of stretched DNA, cover-slips were mounted with 20 µL of a Block-Aid (Invitrogen, ref. B-10710)-YOYO-1 iodide (Molecular Probes, code Y3601) mixture (10000:1 v/v) in order to counter-stain all stretched molecules. Imaging was performed with an inverted automated epifluorescence microscope, equipped with a 40X objective (ImageXpress Micro, Molecular Devices, USA). Length of the YOYO-1-stained DNA fibers were measured with internal software GVlab v0.4.6 (Genomic Vision S.A., Paris, France).

### Results

Fpg enzyme targets a specific subfamily of oxidative damage including 8-oxoguanine, 5-hydroxycytosine, 5-hydroxyuracil, aflatoxin-bound imidazole ring-opened guanine, imidazole ring-opened N-2-aminofluorene-C8-guanine, and open ring forms of 7-methylguanine. The enzyme cleaves the recognized lesion and leaves a nick in the corresponding strand of the DNA. As a result, when two or more lesions are formed really close ("clustered" within 15-20 bases) on opposite strands, the enzymatic treatment generates a double strand break and converts the original fragment into two shorter DNA fragments. The relative amount of "clustered" lesions produced by 4 incremental doses of H₂O₂ was estimated by comparing the final molecular size distributions of the DNA samples following Fpg treatment. Even in the absence of exposure to strong oxidizing agents like H₂O₂, a baseline amount of oxidative lesions is expected to be found in the DNA molecule as oxidation of bases takes place continuously inside the cell due to the presence of free radicals derived from metabolic activities. In order to evaluate the effect produced by the different doses of H₂O₂, the control sample was thus equally submitted to the Fpg treatment. The resulting DNA size distributions are presented in Figure 8*(A)**.* The histograms obtained show a clear, gradual dependence of the DNA size profile on the amount of H₂O₂ used during genotoxic exposure. At high doses of H₂O₂ (10 and 20 mM), the amount of clustered damage present in the molecules is consistent and the stretched strands appear much shorter than in the control sample. To better quantify the PD and the relative increase in the amount of oxidative damage, the distributions were fitted using an exponential model y = Ae^{-x/τ} and the decay constants τ compared as a function of H₂O₂ dose. As depicted by the graph in Figure 8*(B)*, the decay constants appear to decrease linearly with respect to the amount of H₂O₂. The control non-exposed sample is excluded from the linear fit and has a decay constant of 6.1 µm. By comparing the coefficients of the fitting exponentials, it is possible to detect and relatively quantify very small amounts of clustered damage produced by low doses like 1 mM H₂O₂. The method of the invention proves thus much more sensitive with respect to the available techniques. Moreover, the described method maintains high precision over a very large range of genotoxic doses as the histograms are constructed from tens of thousands of measurements at the single molecule level. In order to achieve absolute quantification of damage, it is sufficient to apply a direct quantification method (mass spectroscopy, liquid chromatography etc.) to only one condition and then extrapolate the other ones from the fitting parameters.

In the case of rare events like DSB (60-200 DSB per 2 Gy on 6000 Mbp genome), the size of the original fragments produced by low doses of radiation (30-100 Mbp) is too large to be extracted intact and measured reliably by sizing techniques. Thus, the original PD cannot be evaluated directly. The manipulation of large molecules induces an uncontrolled supplementary fragmentation (Shear DSB), which acts as background noise (non zero-mean). In order to be able to compare measurements profiles, we reduce the contribution of this uncontrolled fragmentation by superposing a controlled and reproducible one, provided by the action of selected Restriction Enzymes (RE). DNA is digested by a set of enzymes producing DNA fragments 30 to 70 kbp in size. Starting from our standard distributions of human genomic DNA, we know that our extraction protocol produces at least 90% of molecules larger than 50 kbp. Fragments reduced to 50 kbp or less after RE digestion are not significantly fragmented during manipulation. To provide an example, if the starting material is human genomic DNA, the restriction enzyme Sma I can be used to produce a population of fragments in this range. As illustrated in Figure 9, to precisely distinguish the RE-DSB (DSB created by the RE) from the Bio-DSB (DSB to be detected, generated in the cell by a genotoxic agent) and the Shear-DSB (DSB generated by shearing during manipulation), we couple specific end-labeling of sticky-DSB generated by the RE. For example, the sticky ends of the human DNA fragments can be labeled with fluorescent dATP by using the Klenow fragment of DNA polymerase. Unlabeled deoxynucleoside triphosphates are added if necessary for incorporation. Optionally, unincorporated label can be removed by precipitation with ethanol. The DNA pool of fragments is stretched on a substrate and the fragments labeled on both extremities are discarded from size measurements. The size distributions are constructed from the measurements of the other fragments and compared to the reference sample, not exposed to the genotoxic agent. The amount of Bio-DSB is then estimated.

### References

### Patents

US 6,130,044: Surfaces for biological reactions, process for preparing them and process for their use. Bensimon D., Bensimon A., Heslot F. Oct 10, 2000
US 6,303,296, US 2006/257910, US 6,054,327: Process for aligning macromolecules by passage of a meniscus and applications. Bensimon D., Bensimon A., Heslot F., Oct 16, 2001.
US 6,225,055: Apparatus for the parallel alignment of macromolecules, and use thereof. Bensimon A., Bensimon D, May 22, 2002.
US 2004/033510: Method for the diagnosis of genetic diseases by molecular combing and diagnostic kit, Bensimon A., Bensimon D., Michalet X., Feb 19, 2004
US 6,789,022: Method for assaying clustered DNA damages, Sutherland B.M., September 7, 2004.
US 7,670,770: Nanochannel arrays and their preparation and use for high throughput macromolecular analysis, Chou S.Y., Cao H., Austin R.H., Yu Z., Tegenfeldt J.O., March 2, 2010.
Provisional Application: Genotoxicity Analysis of DNA using Nanochannels, Cao H., Deshpande P., Austin M., Boyce-Jacino M., 2004.
WO 98/18959: Procédé de diagnostic de maladies génétiques par Peignage Moléculaire et coffret de diagnostic, Bensimon A., Bensimon D., Michalet X., 1998-05-07.
WO 0073503: Utilisation du peignage dans l'identification des origines de replication d'ADN, Bensimon A., Herrick J., Hyrien O., 2000-12-07.
WO 2008/028931: Genomic Morse Code, Lebofsky R., Bensimon A., Walrafen P., 2008-03-13.
WO 2008/121828: Methods of Macromolecular Analysis using Nanochannel arrays, Cao H., Deshpande P., Austin M., Boyce-Jacino M., 2008.
WO 1996/040902: Nucleic Acid Repair Enzyme Methods for Point Mutation and in vitro Mutagenesis, Chirikjian J.G., Collier B. G., Dec. 1996.

### Scientific Publications

Ahmed F. and R. Setlow (1977) "Different rate-limiting steps in excision repair of ultraviolet-and N-acetoxy-2-acetylaminofluorene-damaged DNA in normal human fibroblasts" Proc Natl Acad Sci USA 74: 1548.
Allemand, J.-F., D. Bensimon et al. (1997) "pH-Dependent Specific Binding and Combing of DNA." Biophys. J. 73:2064-2070.
Asaithamby, A. and D. J. Chen (2009) "Cellular responses to DNA double-strand breaks after low-dose gamma-irradiation." Nucl. Acids Res 37: 3912-3923.
Bartsch, H., H. Dally, O. Popanda et al. (2007) "Genetic risk profiles for cancer susceptibility and therapy response." Recent Results Cancer Res. 174:19-36.
Bohr, V. A., C. A. Smith et al. (1985) "DNA repair in an active gene: removal of pyrimidine dimers from the DHFR gene of CHO cells is much more efficient than in the genome overall." Cell 40: 359-369.
Caburet, S., C. Conti, et al. (2005). "Human ribosomal RNA gene arrays display a broad range of palindromic structures." Genome Res 15(8): 1079-1085.
Caldecott, K. W. (2008) "Single-strand break repair and genetic disease." Nature Reviews Genetics 9: 619-631.
Cedervall, B., R. Wong et al. (1995) "Methods for the quantification of DNA double-strand breaks determined from the distribution of DNA fragment sizes measured by pulsed-field gel electrophoresis." Radiat. Res. 144(1):122-130.
Collins, A. R., V. L. Dobson et al. (1997) "The comet assay: what can it really tell us?" Mutat. Res. 75(2):183-193.
Collins, A. R. (2004) "The Comet Assay for DNA damage and repair: principles, applications and limitations." Mol. Biotechnol. 26(3): 249-261.
Conti, C. and A. Bensimon (2002) "A combinatorial approach for fast high-resolution mapping.", Genomics 80: 259-264.
Crew, K. D., M. D. Gammon, M. B. Terry et al. (2007) "Polymorphisms in nucleotide excision repair genes, polycyclic aromatic hydrocarbon-DNA adducts, and breast cancer risk." Cancer Epidemiol. Biomarkers Prev. 16(10): 2033-2041.
de Laat, W. L., N. G Jaspers et al. (1999) "Molecular mechanism of nucleotide excision repair." Genes Dev. 13:768-785.
Dizdaroglu, M. (1984) "The use of capillary gas chromatography-mass spectrometry for identification of radiation-induced DNA base damage and DNA base-amino acid cross-links." J. Chromatogr. 295(1): 103-121. Einhorn, L. H. (2002) "Curing metastatic testicular cancer." Proc. Nat. Acad. Sci. USA. 99: 4592-4595.
Evan, G. I. and K. H. Vousden (2001) "Proliferation, cell cycle and apoptosis in cancer." Nature 411(6835): 342-348.
Fernet, M. and J. Hall (2008) "Predictive markers for normal tissue reactions: Fantasy or reality?" Cancer/Radiothérapie 12(6-7): 614-618.
Filippova, E.M, D.C. Monteleone et al. (2003) "Quantifying damage to double stranded DNA by single molecule laser fluorescence sizing." Biophys. J. 84: 1281-1290.
Finkel, T., M. Serrano et al. (2007), "The common biology of cancer and ageing" Nature 448: 767-774.
Fousteri M. and L. H. Mullenders (2008) "Transcription-coupled nucleotide excision repair in mammalian cells: molecular mechanisms and biological effects." Cell Res. 18(1):73-84.
Gad, S., A. Aurias, et al. (2001). "Color bar coding the BRCA1 gene on combed DNA: a useful strategy for detecting large gene rearrangements." Genes Chromosomes Cancer 31(1): 75-84.
Gad, S., I. Bieche, et al. (2003). "Characterisation of a 161 kb deletion extending from the NBR1 to the BRCA1 genes in a French breast-ovarian cancer family." Hum Mutat 21(6): 654.
Gad, S., V. Caux-Moncoutier, et al. (2002). "Significant contribution of large BRCA1 gene rearrangements in 120 French breast and ovarian cancer families." Oncogene 21(44): 6841-6847.
Gad, S., M. Klinger, et al. (2002). "Bar code screening on combed DNA for large rearrangements of the BRCA1 and BRCA2 genes in French breast cancer families." J Med Genet 39(11): 817-821.
Gerweck, L.E., S. Vijayappa et al. (2006) "Tumor cell radiosensitivity is a major determinant of tumor response to radiation." Cancer Res 66: 8352-5.
Gierlich, J., G. A. Burley, P. M. Gramlich et al. (2006) "Click chemistry as a reliable method for the high-density postsynthetic functionalization of alkyne-modified DNA." Org. Lett. 8(17): 3639-3642.
Hennequin, C., L. Quero and V. Favaudon (2008) "Déterminants et facteurs prédictifs pour la radiosensibilité tumorale." Cancer/Radiothérapie 12(1): 3-13.
Herrick, J., C. Conti, et al. (2005). "Genomic organization of amplified MYC genes suggests distinct mechanisms of amplification in tumorigenesis." Cancer Res 65(4): 1174-1179.
Herrick, J., S. Jun, et al. (2002). "Kinetic model of DNA replication in eukaryotic organisms." J Mol Biol 320(4): 741-750.
Herrick, J., X. Michalet, et al. (2000). "Quantifying single gene copy number by measuring fluorescent probe lengths on combed genomic DNA." Proc Natl Acad Sci U S A 97(1): 222-227.
Herrick, J., P. Stanislawski, et al. (2000). "Replication fork density increases during DNA synthesis in X. laevis egg extracts." J Mol Biol 300(5): 1133-1142.
Hoeijmakers, J. H. J. (2001) "Genome maintenance mechanisms for preventing cancer", Nature 411: 366-374.
Hooper, D. C. (2001) "Mechanisms of action of antimicrobials: focus on fluoroquinolones." Clin. Infect. Dis. 32(Suppl 1):S9-S15.
Ismail, S. M., M. Puppi, S. Prithivirajsingh (2004) "Predicting radiosensitivity using DNA end-binding complex analysis." Clin. Cancer Res. 10(4):1226-1234.
Jamieson, E. R. and S. J. Lippard (1999) "Structure, Recognition, and Processing of Cisplatin-DNA Adducts." Chem Rev. 99(9): 2467-2498.
Kartalou, M., and J. M. Essigmann (2001) "Mechanisms of resistance to cisplatin." Mutat. Res. 478: 23-43. Kelly, C. M. and J. J. Latimer (2005) "Unscheduled DNA synthesis: a functional assay for global genomic nucleotide excision repair." Methods Mol. Biol. 291:303-320.
Khanna, K. K. and S. P. Jackson (2001) "DNA double-strand breaks: signaling, repair and the cancer connection." Nature Genet. 27: 247-254.
Kiyohara, C. and K. Yoshimasu (2007) "Genetic polymorphisms in the nucleotide excision repair pathway and lung cancer risk: a meta-analysis." Int. J. Med. Sci. 4(2): 59-71.
Kunkel, T. A and K. Bebenek (2000) "DNA replication fidelity." Annu. Rev. Biochem. 69: 497-529.
Kurisu, S., T. Miya et al. (2001) "Quantitation of DNA damage by an aldehyde reactive probe (ARP)." Nucleic Acids Res. Suppl 1: 45-46.
Laga A. C. and G. F. Murphy (2009) "The translational basis of human cutaneous photoaging: on models, methods, and meaning." Am. J. of Pathol. 174(2): 357-360.
Larson, E. D., D. W. Bednarski et al. (2008) "High-fidelity correction of genomic uracil by human mismatch repair activities." BMC Mol Biol. 27(9): 94-106.
Latimer, J.J., J.M. Johnson et al. (2010) "Nucleotide excision repair deficiency is intrinsic in sporadic stage I breast cancer" Proc Natl Acad Sci U S A 107: 21725.
Lebofsky, R. and A. Bensimon (2003). "Single DNA molecule analysis: applications of molecular combing." Brief Funct Genomic Proteomic 1(4): 385-396.
Lebofsky, R. and A. Bensimon (2005). "DNA replication origin plasticity and perturbed fork progression in human inverted repeats." Mol Cell Biol 25(15): 6789-6797.
Lebofsky, R., R. Heilig, et al. (2006). "DNA replication origin interference increases the spacing between initiation events in human cells." Mol Biol Cell 17(12): 5337-5345.
Lehmann, A. R. and S. Stevens (1980) "A rapid procedure for measurement of DNA repair in human fibroblasts and for complementation analysis of xeroderma pigmentosum cells." Mutat. Res. 69(1): 177-190.
Limsirichaikul, S., A. Niimi, H. Fawcett et al. (2009) "A rapid non-radioactive technique for measurement of repair synthesis in primary human fibroblasts by incorporation of ethynyl deoxyuridine (EdU)." Nucleic Acids Res. 37(4): e31.
Lindahl, T. and R. D. Wood (1999) "Quality control by DNA repair." Science 286: 1897-1905.
Lisby, S., R. Gniadecki et al. (2005) "UV-induced DNA damage in human keratinocytes: quantitation and correlation with long-term survival." Exp Dermatol. 14(5): 349-355.
MacKay, R. I., A. Niemierko et al. (1998) "Potential clinical impact of normal tissue intrinsic radiosensitivity testing." Radiother. Oncol. 46: 215-216.
Mackay, R. Land J. H Hendry (1999) "The modeled benefits of individualizing radiotherapy patients' dose using cellular radiosensitivity assays with inherent variability." Radiother. Oncol. 50: 67-75.
McCabe, K. M., S. B. Olson et al. (2009) "DNA interstrand crosslink repair in mammalian cells. " J Cell Physiol. 220(3): 569-573.
Michalet, X., R. Ekong, et al. (1997). "Dynamic molecular combing: stretching the whole human genome for high-resolution studies." Science 277(5331): 1518-1523.
Nakamura, J., V. E. Walker et al. (1998) "Highly sensitive apurinic/apyrimidinic site assay can detect spontaneous and chemically induced depurination under physiological conditions." Cancer Res. 58: 222-225. Olive, P. L. (2009) "Impact of the comet assay in radiobiology." Mutat. Res. 681:13-23.
Ostling, O. and K. J. Johanson (1984) "Microelectrophoretic study of radiation-induced DNA damages in individual mammalian cells." Biochem Biophys Res Commun. 123(1): 291-298.
Pasero, P., A. Bensimon, et al. (2002). "Single-molecule analysis reveals clustering and epigenetic regulation of replication origins at the yeast rDNA locus." Genes Dev 16(19): 2479-2484.
Pfeifer, G. P. (1997) "Formation and processing of UV photoproducts: effects of DNA sequence and chromatin environment." Photochem Photobiol. 65(2): 270-283.
Plotz, G., S. Zeuzem et al. (2006) "DNA mismatch repair and Lynch syndrome." J Mol Histol. 37(5-7): 271-283. Rao, V. A., C. Conti, et al. (2007). "Endogenous gamma-H2AX-ATM-Chk2 checkpoint activation in Bloom's syndrome helicase deficient cells is related to DNA replication arrested forks." Mol Cancer Res 5(7): 713-724.
Rogakou, E. P., D. R. Pilch et al. (1998) "DNA double-stranded breaks induce histone H2AX phosphorylation on serine 139." J Biol Chem. 273(10): 5858-5868.
Salic, A. and T.J. Mitchison (2008) "A chemical method for fast and sensitive detection of DNA synthesis in vivo." Proc. Natl. Acad. Sci. USA. 105(7): 2415-2420.
Sattler, U., P. Frit et al. (2003) "Long-patch DNA repair synthesis during base excision repair in mammalian cells" EMBO Re. 4(4): 363-367
Saul, R. L. and B. N. Ames (1986) "Background levels of DNA damage in the population." Basic Life Sci. 38: 529-535.
Schurra, C. and A. Bensimon (2009). "Combing genomic DNA for structural and functional studies." Methods Mol Biol 464: 71-90.
Shrivastav, M., L. P. De Haro et al. (2008) "Regulation of DNA double-strand break repair pathway choice." Cell Res. 18(1): 134-147.
Shuck S. C., E. A. Short et al. (2008) "Eukaryotic nucleotide excision repair, from understanding mechanisms to influencing biology." Cell Res. 18(1): 64-72.
Spitz, M. R., Q. Y. Wei, Q. Dong et al. (2003) "Genetic susceptibility to lung cancer: The role of DNA damage and repair." Cancer Epidemiology Biomarkers & Prevention.12:689-698.
Srivastava, D. K., B. J. Berg et al. (1998) "Mammalian abasic site base excision repair. Identification of the reaction sequence and rate-determining steps." J. Biol. Chem. 273(33): 21203-21209.
Stausbol-Gron, B., J. Overgaard (1999) "Relationship between tumour cell in vitro radiosensitivity and clinical outcome after curative radiotherapy for squamous cell carcinoma of the head and neck." Radiother Oncol 50: 47-55.
Suzuki, K., M. Ojima et al. (2003) "Radiation-induced DNA damage and delayed induced genomic instability." Oncogene 22(45):6988-6993.
Svetlova, M., L. Solovjeva, N. Pleskach et al. (2002) "Clustered sites of DNA repair synthesis during early nucleotide excision repair in ultraviolet light-irradiated quiescent human fibroblasts." Exp. Cell Res. 276(2): 284-295.
Tegenfeldt, J. O., C. Prinz et al. (2004) "The dynamics of genomic-length DNA molecules in 100-nm channels." Proc Natl Acad Sci USA 101(30):10979-10983.
Tornaletti, S. and P. C. Hanawalt (1999) "Effect of DNA lesions on transcription elongation." Biochimie 81: 139-148.
Torres-Roca, J. F., C. W. Stevens (2008) "Predicting response to clinical radiotherapy: past, present, and future directions." Cancer Control. 15(2):151-156.
van der Wees, C., J. Jansen et al. (2007) "Nucleotide excision repair in differentiated cells." Mutat Res. 614(1-2): 16-23.
Vasireddy, R. S., C. N. Sprung et al. (2010) "H2AX phosphorylation screen of cells from radiosensitive cancer patients reveals a novel DNA double-strand break repair cellular phenotype." Br. J. Cancer 102(10): 1511-1518.
Warren, J. J., L. J. Forsberg et al. (2006) "The structural basis for the mutagenicity of 06-methyl-guanine lesions." Proc Natl Acad Sci U S A 103(52): 19701-19706.
White, C. I., S. G. Sedgwick (1985) "The use of plasmid DNA to probe DNA repair functions in the yeast Saccharomyces cerevisiae." Mol. Gen. Genet. 201: 99-106.
Zharkov, D. O. (2008) "Base excision DNA repair." Cell Mol Life Sci. 65(10): 1544-1565.
Zhou, B. B. and S. J. Elledge (2000) "The DNA damage response: putting checkpoints in perspective." Nature 408:433-439.

### Modifications and other embodiments

Various modifications and variations of the described methods, compositions and kits compositions as the concept of the invention will be apparent to those skilled in the art without departing from the claimed invention. Although the invention has been described in connection with specific preferred embodiments, it should be understood that the invention as claimed is not intended to be limited to such specific embodiments.

## Claims

1. A method for detecting the presence or absence of a damaged portion on a nucleic acid, comprising the steps of:
(a) extracting a nucleic acid from a sample, then
(b) stretching the nucleic acid, and
(c) adding a detectable substance to the stretched nucleic acid, which substance positions itself on one or more damaged portions of the stretched nucleic acid by binding to it,
(d) detecting the detectable substance on the stretched nucleic acid,
(e) detecting the presence of a damaged portion on the nucleic acid when said detectable substance is detected and detecting the absence of damaged portion on the nucleic acid when said detectable substance is not detected,
wherein step (c) may be performed either before or after step (b)

2. A method for detecting and quantifying breakages on the backbone on a nucleic acid according to claim 1, wherein step (c) consists of:
(c) adding an enzyme and detectable nucleotides to the stretched nucleic acid, wherein said enzyme labels all DNA 3' free ends with the detectable nucleotides.

3. The method of any one of claims 1 or 2, further comprising diagnosing a disease, disorder or condition by detecting at least one damage on the nucleic acid; and/or further comprising diagnosing recovery from a disease, disorder or condition by detecting at least one damage on the nucleic acid.

4. The method of any of claims 1 to 3, wherein said stretching of the nucleic acids is performed using Molecular Combing.

5. The method of any one of claims 1 to 4, wherein said sample is a tissue sample, or blood, cerebrospinal fluid, synovial fluid, or lymph sample

6. The method of any one of claims 1 to 5, wherein said sample is obtained from a subject who has cancer or who has undergone treatment for cancer.

7. The method of any of claims 1 to 6, wherein said sample is obtained from a subject who has an infectious disease, an autoimmune disease, or an inflammatory disease or condition.

8. The method of any of claims 1 to 7, which further comprises:
(a) hybridizing one or more sequence specific probes specific to one or more specific known positions or regions on the nucleic acid, and, optionally,
(b) determining the location of the detectable nucleotides on the nucleic acid relative to said known positions or regions.

9. A process for determining the effect of a test agent on a nucleic acid sequence in a cell comprising:
contacting the cell with said test agent for a time and under conditions sufficient for it to damage one or more nucleic acids in the cell, and
detecting damages on the nucleic acids of said cell by the method of any of claims 1 to 8, wherein damages may be assessed by comparison to nucleic acid in an otherwise identical cell not exposed to said test agent.

10. The process of claim 9, wherein said test agent is a genotoxic compound or genotoxic ionizing radiation.

11. The method of any of claims 2 to 10 wherein the enzyme is a DNA polymerase capable of forming a detectable nucleotidic tail at the location of the single strand or double strand break.

12. The method of any of claims 2 to 11, wherein the enzyme is TdT polymerase.

13. The method of any of claims 2 to 12, wherein the detectable nucleotides comprise 5'-Ethy-2'-deoxyuridine.
